# EUROPEAN PATENT APPLICATION

(11) **EP 1 695 717 A1**
(43) Date of publication of application: **30.08.2006**
(21) Application number: 05003904.9
(22) Date of filing: 23.02.2005
(51) Int. Cl.: A61K 47/48

(54) **Transport of nano-and macromolecular structures into cytoplasm and nucleus of cells**

(71) Applicant: Ludwig-Maximilians-Universität, 80539 München (DE); Technische Universität München, 80333 München (DE)
(72) Inventor: Rosenecker, Joseph, Dr., 80539 München (DE); Rudolph, Carsten, Dr., 80801 München (DE); Plank, Christian, Dr., 82229 Seefeld (DE); Elfinger, Markus, 82061 Neuried (DE)
(74) Representative: Graf von Stosch, Andreas

(57) **Abstract**

The present invention refers to novel conjugate molecules and their use or the transport of nano- and macromolecular structures into cells and/or the nucleus. More particularly, the present invention refers to conjugate molecules containing a carrier, preferably thiopyridyl moieties, and a cargo moiety. Thereby, the thiopyridyl moiety is bound to the cargo moiety to act as a carrier, particularly for efficient intracellular and/or intranuclear delivery of drugs, nano-or macromolecular structures, etc.. The novel conjugate molecules are provided for the manufacture of a medicament for gene therapy, apoptosis, or for the treatment of diseases such as cancer, autoimmune diseases or infectious diseases.

## Description

The present invention refers to novel conjugate molecules and their use or the transport of nano- and macromolecular structures into cells and/or the nucleus. More particularly, the present invention refers to conjugate molecules containing a carrier, preferably thiopyridyl moieties, and a cargo moiety. Thereby, the thiopyridyl moiety is bound to the cargo moiety to act as a carrier, particularly for efficient intracellular and/or intranuclear delivery of drugs, nano- or macromolecular structures, etc.. The novel conjugate molecules are provided for the manufacture of a medicament for gene therapy, apoptosis, or for the treatment of diseases such as cancer, autoimmune diseases or infectious diseases.

Significant developments have taken place in the last few decades in the development of new pharmaceutically effective molecules. Administration of such pharmaceutically effective molecules, e.g. protein based drugs, frequently requires uptake into cells to enable these molecules to react with their target molecules. The most essential step of such treatments is therefore transport of these molecules into cells crossing the cell membrane and, if required, into the nucleus crossing the nuclear membrane. If pharmaceutically effective molecules fail to enter into the cell or the nucleus, desired reaction of such molecules with their target molecules will not occur and, if at all, no effective therapy is possible.

Cell membranes are lipid bilayers, which typically act as semi-permeable barriers separating the inner cellular environment from the outer cellular (or external) environment. Cell membranes allow only certain molecules to pass while constraining movement of most other molecules, thereby selectively controlling import and export of molecules into/out of cells. Transport of molecules into the cell crossing the cell membrane thus requires specific transport mechanisms, e.g. physiological transport mechanisms.

Physiological transport mechanisms may be divided into passive and active transport mechanisms. Passive transport mechanisms usually require no energy from the cell and are based on movement of molecules along their concentration gradient (i.e. from a region of higher to a region of lower concentration), e.g. by diffusion or osmosis. Typically, passive transport mechanisms are available only for water molecules and a few other small, uncharged, molecules like oxygen, carbon dioxide and small drugs. These molecules diffuse freely in and out of the cell.

In contrast, active transport mechanisms may move molecules against their concentration gradient. Active transport typically occurs via transporter proteins, which use ATP as energy donor. Some transporters bind ATP directly and use energy of its hydrolysis to drive active transport. Other transporters use the energy already stored in the gradient of a directly-pumped ion. Direct active transport of the ion establishes a concentration gradient.

Apart from these physiological transport mechanisms molecules may cross the cell membrane by using so called "cell membrane penetrating peptides", e.g. protein transduction domains (PTD). PTDs are typically small peptides comprising a length of about 20 amino acids and exhibiting a strong basic pH. The arginine rich motif of HIV-1 TAT protein is regarded as a prototype of PTDs. PTDs are capable of crossing cell membranes by non-endocytotic mechanisms. Fusion of PTDs with proteins enable these proteins to cross the cell membrane into the cytoplasm (see. e.g. Snyder, E.L. & Dowdy S.F., Pharm. Res. 21, 389-93 (2004), and Zhao, M. & Weissleder, R., Med. Res. Rev. 24, 1-12 (2004)). Penetrating the cell membrane may thus be enabled by using such systems. Molecules also may cross the cellular membrane by chemically linking PTDs to oligomers (see e.g. Rudolph C. et al., *J*. *Biol. Chem.,* 2003 Mar 28; 278(13):11411-8) or by electrostatic binding of PTDs to DNA or RNA (see e.g. Richard J.P. et al., J. Biol. Chem., 2003, January 3, 278(1): 585-590, 2003). However, PTDs typically degrade in the extracellular environment prior to penetrating the cellular membrane and thus do not allow an efficient and reliable transport of cargos into the cells and the nucleus.

A further problem arises, if molecules act in the nucleus and therefore have to be transported thereto. It is not only required for these molecules to pass the cellular membrane in a first step as discussed above. In addition, these molecules need to penetrate the barrier of the nuclear membrane.

The nuclear membrane represents the second barrier, which is nearly impermeable for most molecules. Transport molecules available in the cytoplasm pass the nuclear membrane typically through nuclear pore complexes (NPCs). These NPCs are large protein structures embedded in the nuclear double membrane. They form cylindrical complexes having an aqueous channel inside. This channel typically comprises a width of about 125 nm and a length of about 150-200 nm. NPCs allow passive and active transport processes. Passive transport processes, e.g. diffusion, enable transport of small molecules, e.g. proteins up to 7 nm in diameter. Transport rate of molecules having a diameter approaching 7 nm is therefore extremely slow, if passive transport mechanisms are used. Therefore, the protein transport rate is decelerated significantly, if the molecule to be transported into the cell has a size of more than 7 nm in diameter. This is to say that small proteins of less than 20-30 kDa effectively diffuse through NPCs, whereas larger molecules, such as BSA, having a molecular weight of about 70 kDa and about 7 nm diameter diffuse very slowly through NPCs (see e.g. Dorlich & Kutay, Annu. Rev. Cell Dev. Biol. 15, 607-60 (1999)). Larger molecules therefore use different mechanisms for entering the nucleus via NPCs, e.g. by using physiological nuclear transport signals in an active transport mechanism. The aqueous channel inside the NPC may then be expanded to about 40 nm in diameter.

Nuclear transport signals as physiologically required for active transport into the nucleus are recognised by specific transport receptors which directly interact with components of the NPC and promote the actual translocation process. To accomplish multiple rounds of transport, these transport receptors shuttle continuously between nucleus and cytoplasm. Physiologically occurring transport receptors can be divided into two groups depending on the direction in which they transport a substrate: importins transfer a substrate into the nucleus whereas exportins carry their substrate to the cytoplasm. Transport receptors enable unidirectional transport of a specific substrate. Hence, accumulation of an import substrate in the nucleus or of an export substrate in the cytoplasm has to be regulated in a compartment-specific manner. The small GTPase Ran is a determinant of this compartimental "identity" (see e.g. Dorlich & Kutay (1999), supra). In the nucleus Ran is maintained in its GTP-bound form by a chromatin-associated nucleotide exchange factor (RanGEF). In the cytoplasm, RanGTP is readily converted to RanGDP by virtue of the RanGTPase activating protein, RanGAP. This leads to a steep gradient in the concentration of RanGTP across the NPC. Substrate binding to transport receptors appears to be directly regulated by RanGTP binding. Import receptors, e.g. importin-β, bind their substrate only in the absence of RanGTP (cytoplasmic environment). They release their substrate upon RanGTP binding (nuclear environment). The dimeric complex of RanGTP/importin-β is then transported into cytoplasm and recycled.

Binding of substrate via importin-β typically occurs via so called nuclear localization sequences (NLSs). They consist of one or more clusters of basic amino acids and are part of the substrate to be imported. Importin-β may bind its substrate directly via its NLS. Alternatively, binding of a substrate via its NLS to importin-β may also be mediated via importin-α. Importin-α then serves as an adapter to importin-β. The so formed dimeric or trimeric complex passes the nuclear membrane by using importin-β as a transporter. In the nucleus, the dimeric or trimeric complex releases its substrate into the nucleoplasm upon binding RanGTP as indicated above. Once in the nucleus, the import receptor importin-β void of its cargo and its adaptor, importin-α, are exported and recycled in the cytoplasm to allow multiple rounds of import.

Cargo molecules need to be transported into the nucleus, if cargo molecules are present in the cytoplasm but act in the nucleus. Therefore, active transport mechanisms may be applied utilizing the above mentioned NLSs as a component. Cargo molecules may be pharmaceutically effective molecules such as proteins or nucleic acids (see e.g. Munkonge et al., Adv Drug Deliv. Rev. 55 (2003), 749-60).

Transport mechanisms utilizing NLS sequences typically focus on the transport of cargo proteins into the nucleus. Therefore, fusion proteins are typically formed on the basis of an NLS sequence and a cargo protein. Such a fusion protein typically allows a cargo protein to enter into the nucleus via NPCs. However, this method does not allow proteins to pass the lipid bilayer of the cell membrane into the cytoplasm, which is a prerequisite for use of the NLS sequence as carrier moiety of therapeutically effective cargo. Therefore, extracellular application of NLS fusion proteins results in non-efficient transfection of cells, and, consequently, in a non-efficient transport into the nucleus.

If used in gene transfer experiments, cargo proteins may be encoded by respective nucleic acids, which then have to be transfected into living cells. However, transfection of such nucleic acids into living cells also requires crossing the lipid bilayer of cell membranes, which, in most cases, is accomplished to an unsatisfactory extent. Alternatively to the above, state-of-the-art methods also use pharmaceutically active (complex) compositions as cargo moieties. These compositions are usually administered extracellularly and have to cross lipid bilayers of cell membranes, and if necessary, even nuclear membranes. According to recent developments these pharmaceutically active (complex) compositions are preferably formulated as liposomal (see e.g. Chesnoy and Huang, Annu. Rev. Biomol. Struct. 2000, 29:27-47) or polymer based (see e.g. Merdan et al, Adv. Drug Deliv. Rev. (54), 2002, 715-58) compositions.

Liposomal compositions enhance solubilities of pharmaceutical compositions showing only low or extremely low solubitity per se. They may interact with the cell membrane and thereby enable the pharmaceutical compositions to be taken up by target cells. However, liposomal compositions are typically capable to cross the outer cell membrane, but are not channeled through the nuclear membrane into the nucleus. Additional to the above, liposomal compositions are typically exposed to degradation by metabolic processes subsequent to administration. In order to prevent such degradation, liposomal compositions may further be treated with e.g. polyethylene glycol (PEG), which is incorporated into the liposomal membrane. This protection is known as "stealth effect", since incorporation of PEG-chains prevents incorporation of the liposomal composition by the reticulo endothelial system. However, PEGylation significantly reduces interaction of the composition with the cell membrane, and thus significantly reduces uptake of a pharmaceutical composition into cells.

Summarizing the above, transport of different cargo moieties into cells crossing cellular and nuclear membranes by using state-of-the-art methods leads to unsatisfactory transport rates. Usually, no effective treatment may be obtained upon using such methods. Consequently, there is a need in the art to provide systems, which allow to transport cargo moieties such as nucleic acids, fusion proteins, etc. into cells crossing cell and/or nuclear membranes.

It is the object of the present invention to provide carrier moieties for transporting different types of cargo moieties into cells enabling the cargo moiety to cross the cell membrane and, advantageously, the nuclear membrane.

This object is solved by a conjugate molecule according to the present invention.
Inventive conjugate molecules according to the present invention comprise at least one first portion (I) as a carrier moiety and one second portion (II) as a cargo moiety.

**Portion (I)** of the inventive conjugate molecule, the carrier moiety, is preferably a disulfide containing moiety as defined by general formula (I) below: 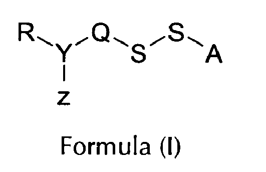 wherein:
- R: is portion (II), if covalently bound to portion (I), or a (substituted or non substituted) pyridyl moiety, N-maleimido moiety, N-succinimidyl moiety, (C₆H₃)(OH)N₃; C(N₂)CX₃; NHNH₂Cl; p-Nitrophenyl; or A as defined below, or not present;
- Q: is selected from the group consisting of:
a bond or (CH₂)ₙ, (CH₂)ₙC(O)NH(CH₂)ₙ, (CH₂)ₙC(O)NHNH₂-HCl, (CH₂)ₙC(O)NH(CH₂)ₙNH, (CH₂)ₙNHC(O)(CH₂)ₙ, (C₆H₄)CH(CH₃), (CH₂)ₙ(C₆H₄), (C₆H₈)CH, (CH₂)ₙNHC(O)(C₆H₄)CH(CH₃), or a or a branched or linear C₁-C₁₀- (hetero)alkyl, C₁-C₁₀-(hetero)alkenyl, C₁-C₁₀-(hetero)cycloalkyl, C₁-C₁₀₋(hetero)cycloalkenyl, C₁-C₁₀-alkoxy, C₁-C₁₀-alkenoxy, C₁-C₁₀-acyl moiety or a C₁-C₁₀-cycloalkyl moiety, substituted or non substituted C₁-C₁₄-aryl, C₁-C₁₄₋heteroaryl, arylalkyl, arylalkenyl, C₅₋₁₄-aryloxy, heteroarylalkyl, heteroarylalkenyl, heterocycloalkyl, heterocycloalkenyl, or heteroaryloxy moiety;
- Y: is a bond; or C, CH, C-O-, -O-C-, or A as defined below;
- Z: is H, O, OH, OCH₃, COOH, COOCₙH₂ₙ₊₁, CO, CH₃, (CH₂)ₙCH₃, (CH₂)ₙOH, NH₂, NO, NO₂, NOH, N, NHNH₂, NC, CN, S, SH SO₂; or Z is not present;
- A: is selected from the group consisting of
CH₂X, or a (substituted or non substituted) C₁-C₁₄-aryl, C₁-C₁₄-heteroaryl, arylalkyl, arylalkenyl, C₅₋₁₄-aryloxy, heteroarylalkyl, heteroarylalkenyl, heterocycloalkyl, heterocycloalkenyl, or heteroaryloxy moiety, particularly a (substituted or non substituted) pyridyl moiety, N-maleimido moiety, or a N-succinimidyl moiety;
wherein n = 0-10, and X = F, Cl, Br, I, CN or CO.

In a preferred embodiment, portion (I) of the inventive conjugate molecule may be a moiety as defined by formulas (Ia), (Ib) or (Ic) below: 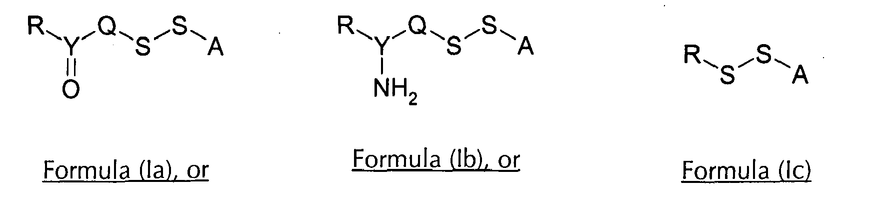 wherein A, Q, R, Y and Z are as defined above, more preferably, wherein A and R are selected from a (substituted or non substituted) pyridyl moiety, N-maleimido moiety or N-succinimidyl moiety.

Alternatively, portion (I) of the inventive conjugate molecule, the carrier moiety, may be a moiety as defined by general formula (II) below: 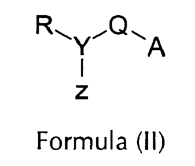 wherein, A, Q, R, Y and Z are as defined above.

Portion (I) of the inventive conjugate moiety is typically covalently coupled to portion (II) as defined below and as indicated by general formula (I). Portion (I) of the inventive conjugate moiety may be provided along with any chemically reactive moiety which allows to react portion (I) with portion (II) as defined below. Preferably, this chemically reactive moiety is a suitable leaving moiety (e.g. R or A as defined above), which allows to covalently link portion (I) to portion (II) and which is removed subsequent to reaction. More preferably, such a leaving moiety may be a N-succinimidyl moiety, a pyridyl moiety, e.g. pyridine-2-thione, p-nitrophenol, an acylhalogenide...., etc.

Alternatively the chemically reactive moiety is a moiety (e.g. R or A as defined above), which allows to covalently link portion (I) with portion (II), but which is not removed subsequent to reaction. Such reaction may occur when binding portion (I) to portion (II) via an addition reaction, e.g. to an unsaturated moiety, e.g. an epoxide containing moiety or an unsaturated hydrocarbon moiety, etc..

An alkyl moiety as defined herein is meant to comprise saturated linear as well as branched or cyclic C₁-C₁₀, hydrocarbon structures as well as combinations thereof. "Lower alkyl moiety" typically refers to alkyl groups containing 1 to 6 carbon atoms. Examples of lower alkyl groups include methyl-, ethyl-, propyl-, isopropyl-, butyl-, sand t-butyl-moieties and similar structures. Cyclic alkyl moieties represent a subgroup of alkyl moieties and include cyclic carbon atom moieties comprising 3 to 14 carbon atoms. Examples of cyclic alkyl moieties include cyclopropyl, cyclobutyl, cyclopentyl, norbornyl moieties and similar structures.

An alkenyl moiety according to portion (I) of the inventive conjugate molecule typically comprises non-saturated linear as well as branched or cyclic C₁-C₁₀, hydrocarbon structures as well as combinations thereof. "Lower alkenyls" typically refer to alkyl groups containing 1-6 carbon atoms. Examples of lower, alkenyl groups include methenyl-, ethenyl-, propenyl-, isopropenyl-, butenyl-, s- and t-butenyl-moieties and similar structures. Cyclic alkenyles represent a subgroup of alkenyles and include cyclic carbon atom moieties comprising 3 to 14 carbon atoms. Examples of cyclic aylkenyles include cyclopropenyl, cyclobutenyl, cyclopentenyl and similar structures.

An heteroalkyl or heteroalkenyl moiety according to the present invention typically comprises an alkyl or alkenyl as defined above, whereby one or more carbon atoms have been substituted with a heteroatom, such as oxygen, nitrogen, sulphur, etc..

Alkoxy- or alkoxyl moieties according to the present invention typically refer to structures having saturated linear, branched or cyclic C₁-C₁₀ hydrocarbon structures as well as combinations thereof, being linked with the hydrocarbon structure via an oxygen atom. Examples include methoxy-, ethoxy-, propoxy-, isopropoxy-, cyclopropyloxy-, cyclohexyloxy- and similar moieties. "Lower alkoxy-" or "lower alkoxyl-" moieties refer to moieties comprising 1 to 4 carbon atoms.

Alkenoxy- or alkenoxyl moieties according to the present invention refer to structures having non-saturated linear, branched or cyclic C₁-C₁₀ hydrocarbon structures as well as combinations thereof , being linked with the hydrocarbon structure via an oxygen atom. Examples include methenoxy-, ethenoxy-, propenoxy-, isopropenoxy-, cyclopropylenoxy-, cyclohexylenoxy- and similar moieties. "Lower alkenoxy-" or "lower alkenoxyl-" moieties refer to moieties comprising 1 to 4 carbon atoms.

Aryl and heteroaryl moieties according to the present invention typically refer to structures having a 5 or 6 membered aromatic or heteroaromatic ring system comprising 0 to 3 heteroatoms selected from O, N, or S; a bicyclic 9 or 10 membered aromatic or heteroaromatic ring system comprising 0 to 5 heteroatoms selected from O, N, or S; or a tricyclic 13 or 14 membered aromatic or heteroaromatic ring system comprising 0 to 7 heteroatoms selected from O, N, or S. Aromatic 6 to 14 membered ring systems include e.g. benzene, naphthalene, indane, tetraline, und fluorene and 5 to 10 membered aromatic or heteroaromatic ring systems include e.g. imidazole, pyridine, indole, thiophene, benzopyranone, thiazole, furane, benzimidazole, chinoline, isochinoline, chinoxaline, pyrimidine, pyrazine, tetrazole und pyrazole.

Arylalkyl moieties according to the present invention typically refer to an alkyl moiety as defined above, wherein the alkyl moiety is bound to an aryl as defined above. Examples of arylalkyl moieties according to the present invention include e.g. benzene, phenethyl, etc.. Heteroarylalkyl refers to an alkyl moiety as defined above, being bound to an heteroaryl moiety as defined above. Heteroarylalkenyl refers to an alkenyl moiety as defined above, being bound to an heteroaryl moiety as defined above. Examples include e.g. pyridinylmethyl, pyrimidinylethyl, etc..

A heterocyclic moiety according to the present invention typically refers to a cycloalkyl- or aryl moiety as defined above, wherein one or two carbon atoms are substituted with oxygen, nitrogen or sulphur.

Heteroaryl moieties typically represent a subgroup of heterocyclic moieties. Examples of heterocyclic moieties include e.g. pyrrolidine, pyrazole, pyrrole, indole, chinoline, isochinoline, tetrahydroisochinoline, benzofurane, benzodioxane, benzodioxole (typically referred to as methylenedioxybenzene, if occurring as substituent), tetrazole, morpholine, thiazole, pyridine, pyridazine, pyrimidine, thiophene, furane, oxazole, oxazoline, isoxazole, dioxane, tetrahydrofurane, etc..

Acyl moieties according to the present invention typically refer to moieties having 1 to 6 carbon atoms showing the structure RCO, wherein R may be any of the afore mentioned moieties. Exemplary acyl moieties are e.g. methanoyl-, acetyl-, ethanoyl-, propanoyl-, butanoyl-, malonyl-, benzoyl-, etc..

Any alkyl, alkenyl, heteroalkyl, heteroalkenyl, alkoxy, alkenoxy, acyl, cycloalkyl, cycloalkenyl, aryl, heteroaryl, arylalkyl, arylalkenyl, aryloxy, heteroarylalkyl, heteroarylalkenyl, heterocycloalkyl, heterocycloalkenyl, carboxamido-, acylamino-, amidino-, or heteroaryloxy moiety as defined herein, may be substituted. If substituted, preferably 1 to 10 H atoms are typically substituted with H, N, SO₃⁻, SO₃H, SO₃Na, SH, O, OH, OCH₃, X, CX₃, CHX₂, CH₂X, OCX₃, CN, CO, COOH, NH, NH₂, NO, NO₂, N₃, or NOH or a branched or linear C₁-C₁₀-alkyl, C₁-C₁₀₋alkenyl, C₁-C₁₀-heteroalkyl, C₁-C₁₀-heteroalkenyl, C₁-C₁₀-alkoxy, C₁-C₁₀-alkenoxy, C₁-C₁₀-acyl, C₁-C₁₄-cycloalkyl, C₁-C₁₄-cycloalkenyl, C₁-C₁₄-aryl, C₁-C₁₄-heteroaryl, arylalkyl, arylalkenyl, C₅₋₁₄-aryloxy, heteroarylalkyl, heteroarylalkenyl, heterocycloalkyl, heterocycloalkenyl, carboxamido-, acylamino-, amidino-, or an heteroaryloxy moiety, as defined above.

Furthermore, A, Q or R, as defined herein, may be substituted. If substituted, A, Q or R are typically substituted with H, N, SO₃⁻, SO₃H, SO₃Na, SH, O, OH, OCH₃, X, CX₃, CHX₂, CH₂X, OCX₃, CN, CO, COOH, NH, NH₂, NO, NO₂, N₃, or NOH.

An halide X typically comprises F, Cl, Br, und I. Alternatively, pseudohalides CN or CO may be used as X.

Any molecule of formula (I) may comprise one or more asymmetric centres. Molecules of formula (I) therefore may form enantiomers, diastereomers, and further stereomeric forms. According to terms of absolute stereochemistry these stereomeric forms are preferably indicated by terms (R) or (S). The present invention comprises such possible isomeric forms as well as their pure or racemic forms. Optically active (R)-or (S)-isomers may be purified by using synthosan or chirale reagents or by using state-of-the-art separation methods. E- and Z-isomers are also encompassed by molecules of formula (I), if these molecules comprise olefinic double bonds or other geometric centres of asymmetry. Similarly, any tautomeric form is encompassed.

Exemplary examples of moieties according to formula (I) may be selected from the following, without_being limited thereto:

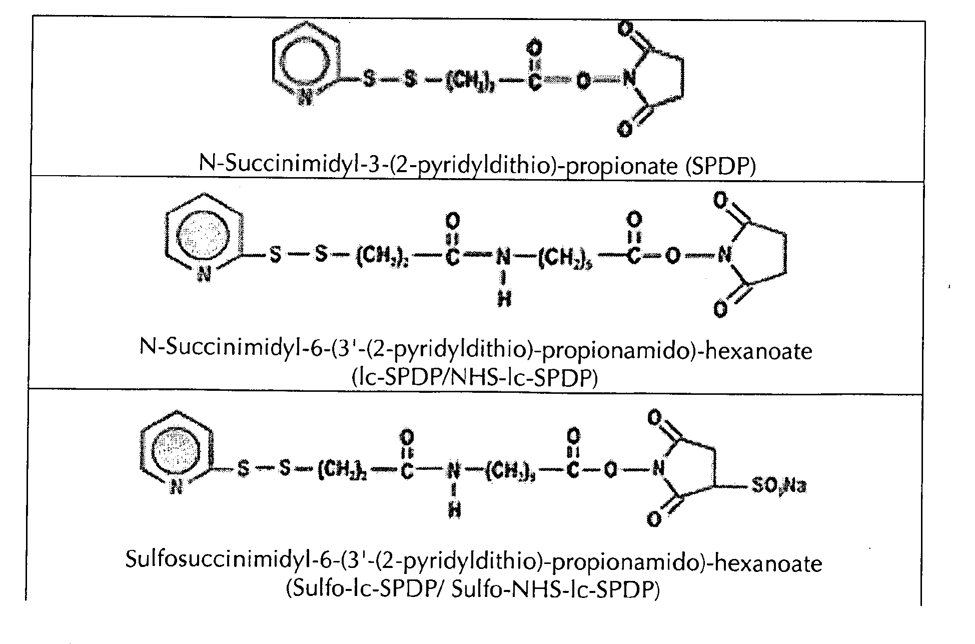

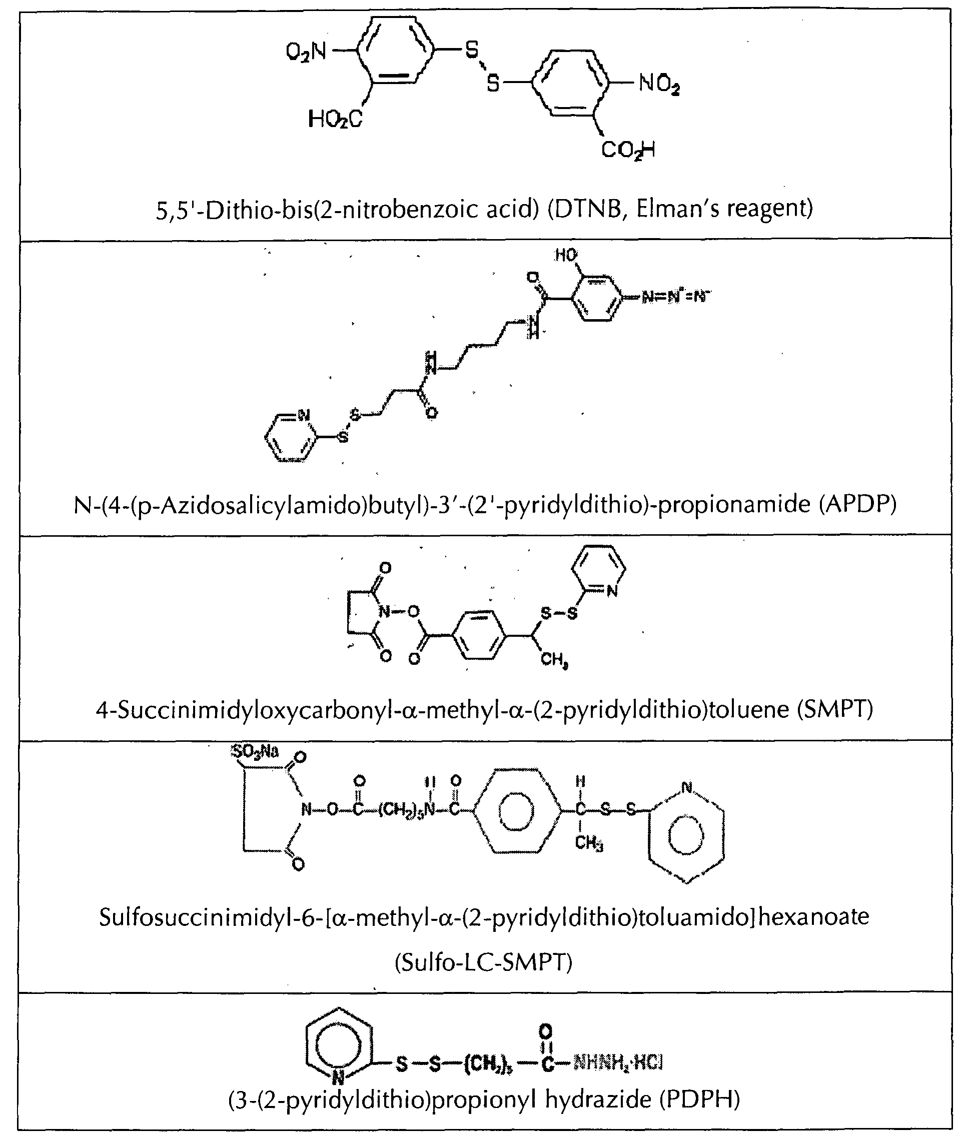

Exemplary examples of moieties according to formula (II) may be selected from the following, without being limited thereto:

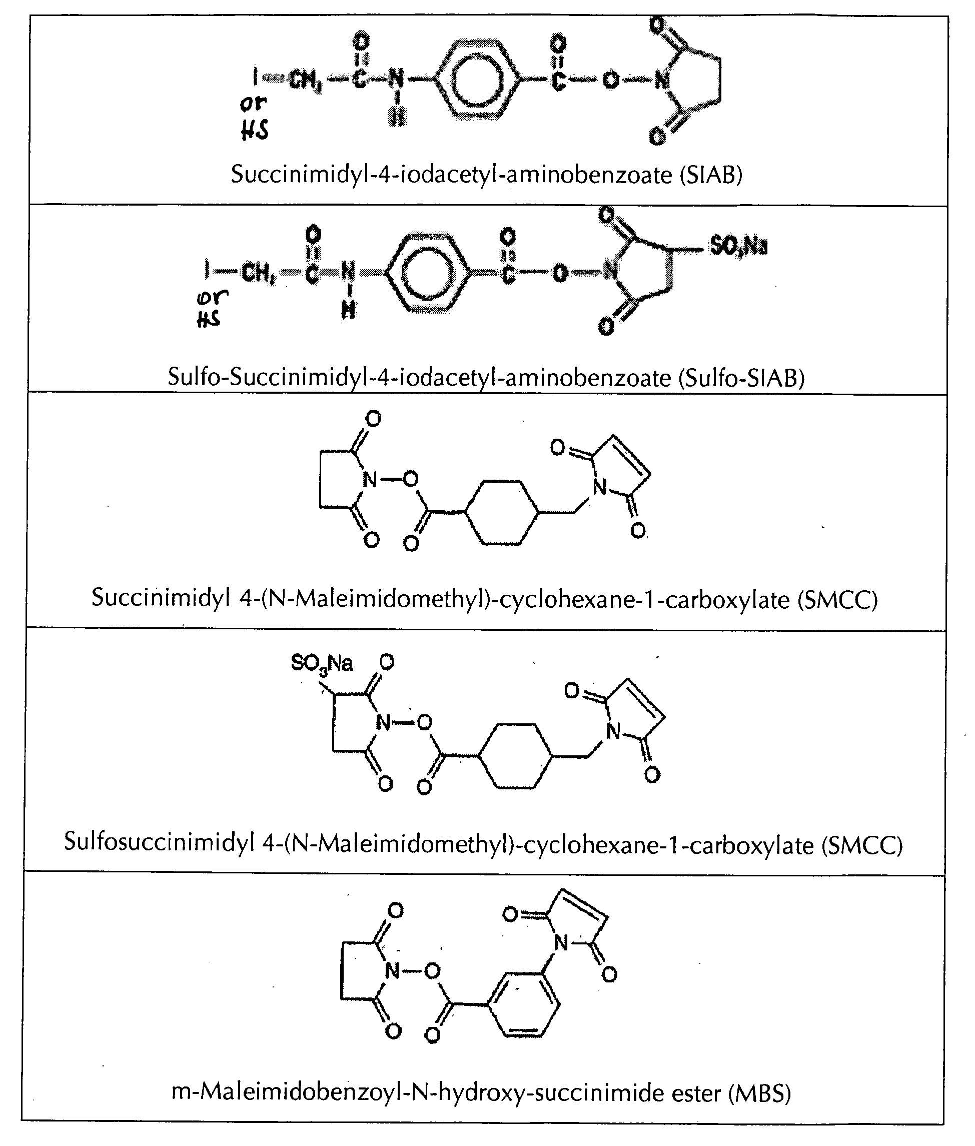

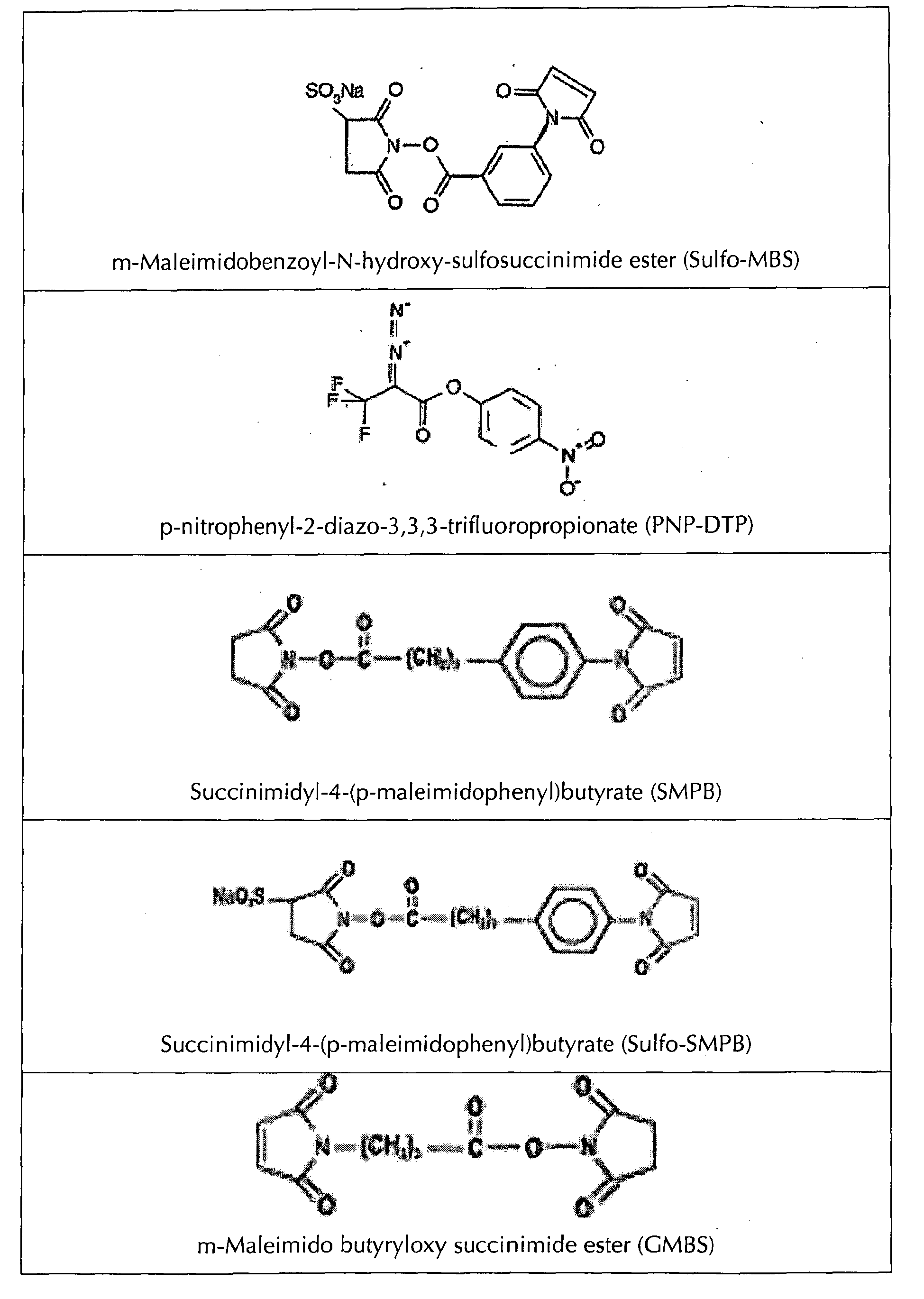

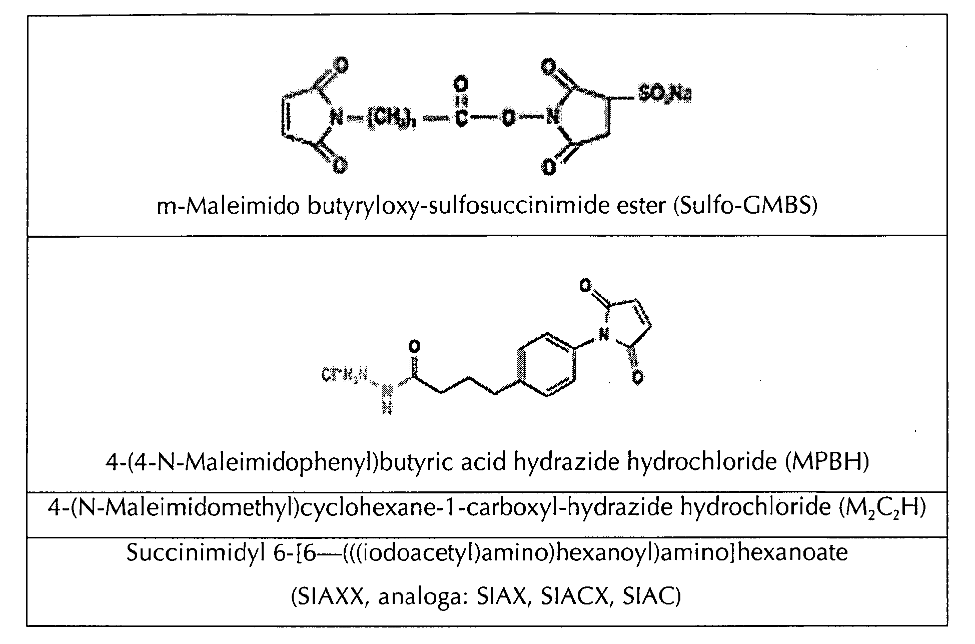

Portion (II) of the inventive conjugate molecule typically represents a biologically active cargo moiety but may also be a biologically non-active cargo-moiety, such as a polymer, e.g. used for formulating a biologically active compound in a complex composition. Portion (II) as a biologically active cargo moiety is typically selected from any moiety capable of interacting with cellular components and/or with physiologic ally occurring nuclear components contained in the nucleus. Such moieties include e.g. proteins, in particular apoptosis inducing or apoptosis related factors or antibodies, nucleic acids, anti-tumor drugs, e.g. cytostatic agents, therapeutically active organic or inorganic molecules, (complex) compositions comprising therapeutically active organic or inorganic molecules, liposomal compositions, polymers, drug carriers, excipients, etc..

Proteins used as portion (II) may be any prokaryotic, eukaryotic protein or artificial protein, preferably a viral, bacterial, plant, human or animal protein including toxic proteins therefrom. It may be provided in its full length form or as a partial sequence, e.g. domain. Proteins used as portion (II) may occur in the cytoplasm and/or the nucleus. Proteins preferably occurring in the cytoplasm comprise for instance one or more interaction domains of a protein, such as the SH1, SH2 or SH3-domain of a protein, or, alternatively one or more multimerisation domains. Proteins used as portion (II) which act in the nucleus and/or the cytoplasm are e.g. growth factors, e.g. epidermal growth factor (EGF), platelet-derived growth factor (PDGF), fibroblast growth factors (FGFs), transforming growth factor-α (TGF-α), transforming growth factors-β (TGFs-β), Erythropietin (Epo), insuline-like growth factor-I (IGF-I), insuline-like growth factor-II (IGF-II), Interleukin-1 (IL-1), Interleukin-2 (IL-2), Interleukin-6 (IL-6), Interleukin-8 (IL-8), tumor necrosis factor-α (TNF-α), tumor necrosis factor-β (TNF-β), Interferon-γ (IFN-γ), colony stimulating factors (CSFs), etc., or a protein involved in transduction of G-protein coupled signals or a matrix or cellular skeletal protein, or fragments thereof. Proteins used as portion (II) which preferably act in the cytoplasm may further be derived from ubiquitine, ubiquitine related proteins or proteins sharing a significant similarity with ubiquitine (e.g. more than 75% sequence identity with the amino acid sequence of ubiquitine), or other proteins involved in intracellular decomposition processes or apoptosis, or fragments thereof. Proteins used as portion (II), which comprise DNA binding domains, e.g. Zinc finger domains, leucine zipper domains, e.g a GCN4 basic leucine zipper domain, homeo domains, etc., as well as proteins derived from the family of transcription factors, e.g. TFIIA, TFIIB, TFIID/TATA binding protein, TFIIE, TFIIF and TFIIH (CTD kinase), etc. preferably interact with proteins being localized the nucleus. Proteins used as portion (II) also may comprise amino acids in their sequence, which render these proteins toxic, e.g. fluorite amino acids.

Apoptosis inducing or apoptosis related factors used as portion (II) may act in the cytoplasm as well as in the nucleus and include e.g. proteins and/or factors selected from or associated with IFF, Apaf, e.g. Apaf-1, Apaf-2, Apaf-3, or APO-2(L), APO-3(L), Apopain, Bad, Bak, Bax, Bcl-2, Bcl-x_{L}, Bcl-x_{S},bik, CAD, calpains, caspases, e.g. caspase-1, caspase-2, caspase-3, caspase-4, caspase-5, caspase-6, caspase-7, caspase-8, caspase-9, caspase-10, caspase-11, granzyme B, or ced-3, ced-9, ceramide, c-Jun, c-Myc, CPP32, crm A, cytochrome C, D4-GDP-DI, Daxx, DcR1, DD, DED, DISC, DNA-PK_{CS}, DR3, DR4, DR5, FADD/MORT1, FAK, Fas, Fas-ligand CD 95/fas (receptor), FLIP, Fodrin, fos, G-Actin, Gas-2, gelsoline, glucocorticoid/ glucocorticoid receptor, granzyme A/B, hnRNPs C1/C2, ICAD, ICE, JNK, Lamin A/B, MAP, MCL-1, Mdm-2, MEKK-1, NEDD, NF-κB, NuMa, p53, PAK-2, PARP, perforin, phosphatidylserine, PITSLRE, PKC δ, pRb, preseniline, price, RAIDD, Ras, RIP, sphingomyelinase, SREBPs, TNF-α, TNF-α receptor, TRADD, TRAF2, TRAIL, e.g. TRAIL-R1, TRAIL-R2, TRAIL-R3, or transglutaminase, U1-70 kDa snRNP, YAMA.

Antibodies also may be used as portion (II) of the inventive conjungate. Antibodies may act in the cytoplasm as well as in the nucleus. Antibodies used as portion (II) may act intracellularly as well as in the nucleus. Accordingly, antibodies may have to penetrate the cellular and, if necessary, the nuclear membrane. Antibodies may be directed to specific targets, e.g. structures, antigens, proteins, etc. in the cytoplasm or in the nucleus. According to the present application, the term "antibody" comprises monoclonal antibodies, polyclonal antibodies, particularly polyclonal monospecific antibodies (i.e. antibodies with different variable regions, which however all recognize a specific epitope), as well as chimeric antibodies, idiotypic antibodies, anti-idiotypic antibodies, (anti-)anti-idiotypic antibodies, and genetically manipulated antibodies that are all present in bound or soluble form and may - if appropriate - be labelled by "markers" (for example fluorescence marker, gold marker, coupled enzymes). The term "antibody" in the meaning of the present invention typically refers to full-length antibodies of the afore mentioned antibodies or fragments thereof. A "full-length" (monoclonal) antibody in the meaning of the present application may be any of the above mentioned inventive antibodies in its full-length form. A full-length antibody of the present invention typically comprises both the domains of the heavy chain and the light chain. The heavy chain of the inventive antibody includes domains C_{H}1, C_{H}2 or C_{H}3 of the constant region and the variable heavy (V_{H}) immunoglobulin domain. The light chain of the inventive antibody includes the variable light immunoglobulin domain (V_{L}) and the constant light immunoglobulin domain (C_{L}). Antibodies, not containing all the aforementioned domains or regions of an antibody, are fragments of antibodies within the meaning of the present invention.

Fragments of antibodies may be produced by recombinant DNA techniques or by enzymatic or chemical cleavage of intact antibodies. Fragments of antibodies include, inter alia, Fab,Fab', F(ab') 2, Fv, dAb, and complementarity determining region (CDR) fragments, single-chain antibodies (scFv), chimeric antibodies, diabodies and polypeptides that contain at least a portion of an immunoglobulin that is sufficient to confer specific antigen binding. An Fab fragment is a monovalent fragment consisting of the VL, VH, CL and CH1 domains; a F(ab') 2 fragment is a bivalent fragment comprising two Fab fragments linked by a disulfide bridge at the hinge region; a Fd fragment consists of the VH and CH1 domains; an Fv fragment consists of the VL and VH domains of a single arm of an antibody; and a dAb fragment (Ward et al., Nature 341: 544-546,1989) consists of a VH domain. A single-chain antibody(scFv) is an antibody in which a VL and VH regions are paired to form a monovalent molecules via a synthetic linker that enables them to be made as a single protein chain (Bird et al., Science 242: 423-426 (1988) and Huston et al., Proc. Natl. Acad. Sci. USA 85: 5879-5883, (1988)). Diabodies are bivalent, bispecific antibodies in which VH and VL domains are expressed on a single polypeptide chain, but using a linker that is too short to allow for pairing between the two domains on the same chain, thereby forcing the domains to pair with complementary domains of another chain and creating two antigen binding sites (see, e. g., Holliger et al., Proc.Natl. Acad. Sci. USA 90: 6444-6448 (1993), and Poljak et al., Structure 2: 1121-1123 (1994)).

Antibodies used as portion (II) of the inventive conjugate may pertain to one of the following immune globulin classes: IgG, IgM, IgE, IgA, GILD and, if applicable, a subclass of the aforementioned classes, such as the subclasses of the IgG or their mixtures. IgG and its subclasses such as IgG1, IgG2, IgG2a, IgG2b, IgG3 or IgGM are preferred. The IgG subtypes IgG1/k or IgG2b/k are specifically preferred.

Moreover, antibodies used as portion (II) may be proteins or possibly other structures produced by vertebrates or by artificial production methods, that bind with high affinity to a determined surface conformation (epitope) of an antigen, i.e. of another molecule, preferably mono- or polyclonal (partial) structures of the above mentioned immune globulins or also polyclonal monospecific antibodies. Typically, such antibodies contain at least the variable part of immune globulins, and, as the case may be, at least one domain of the constant domain of immune globulins, too.

Nucleic acids used as portion (II) in the inventive conjugate may also act in the cytoplasm as well as in the nucleus. Nucleic acids used as portion (II) may be derived from any biological or synthetic source or may be contained in nucleic acid libraries or databases, e.g. databases for genomic DNA, artificial chromosomes, mini chromosomes, subgenomic DNA, cDNA, synthetic DNA sequences, DNA-enzymes, RNA sequences, siRNA, ribozymes, e.g. hammerhead ribozymes, or may directly be derived from such sequences or combinations thereof. Nucleic acid sequences preferably occurring in the nucleus may be selected from genomic DNA, subgenomic DNA, cDNA, synthetic DNA sequences, DNA-enzymes and may encode any of the peptides or proteins as defined above. Preferably, the reading frame of such nucleotide sequences as portion (II) is not interrupted by a stop codon. Nucleic acids used as portion (II) and preferably occurring in the nucleus further may comprise anti-sense oligonucleotides, preferably anti-sense oligonucleotides suitable to enhance or suppress transcription of a specific cellular or nuclear protein. Nucleic acid sequences preferably occurring in the cytoplasm as portion (II) are e.g. RNA sequences, siRNA, ribozymes, e.g. hammerhead ribozymes, or may be derived from such sequences or combinations thereof.

Portion (II) may also contain cytostatics or drugs related to tumor treatment. It is preferred to select such cytostatics or drugs of the platin (derivative) and taxol classes. In particular, cytostatics or drugs are selected from the group consisting of, for example, cisplatin, satraplatin, oxaliplatin, carboplatin, nedaplatin, chlorambucil, cyclophosphamid, mephalan, azathioprin, fluorouracil, mercaptopurin, methrexat, nandrolon, aminogluthemid, medroxyprogesteron, megestrolacetate, procarbazin, docetaxel, paclitaxel, epipodophyllotoxin, podophyllotoxin, vincristine, docetaxel, daunomycin, doxorubicin, mitoxantrone, topotecan, bleomycin, gemcitabine, fludarabine, and 5-FUDR.

Alternatively to the above, portion (II) of the inventive conjugate may also be a (biologically not active) polymer, such as dextran, pluronics, e.g. polyethyleneoxide (PEO), polypropyleneoxide (PPO), polyethyleneglycol (PEG), poylethyleneimine (PEI), poly[N-(2-hydroxypropyl)methacrylamide] (PHPMA), starch, poly-L-lysine (pLL), poly-R-lysine (pLR), Chitosan, poly-glutamic acid, etc.. Such conjugate polymers, containing portions (I) and (II), may be used as adjuvants.

The above conjugate polymers may be a component of a (complex) pharmaceutical composition. Various formulation alternatives may be considered, when preparing such a (complex) pharmaceutical composition.

In a first alternative, the above conjugate polymers may be used to formulate the pharmaceutical composition. Conjugate polymers in this context are typically polymers as defined above (portion (II)), which already have been reacted with portion (I) as defined herein. In more detail, such a pharmaceutical composition is typically prepared by reacting the conjugate polymer with a therapeutically active compound or a mixture of therapeutically active compounds such as to obtain the desired pharmaceutical composition. Then, the conjugate polymer used in the formulation, enables the therapeutically active compound or the mixture of therapeutically active compounds to penetrate the cell membrane and advantageously the nuclear membrane.

In a second alternative, the therapeutically active compound or a mixture of therapeutically active compounds may be formulated in a first step by using a polymer as defined above, wherein the polymer (portion (II)) has not yet been reacted with portion (I), the cargo moiety. A pharmaceutical composition obtained thereby includes any commercially available or otherwise readily prepared pharmaceutical composition, preferably formulated by using polymers as defined above. In a second step, the polymers, contained in such a pharmaceutical composition, are reacted with a portion (I) as defined herein, enabling the final, pharmaceutical composition, to penetrate the cell membrane and advantageously the nuclear membrane.

The pharmaceutical composition as mentioned above, may further be a liposomal composition. Liposomal compositions are typically formed by components such as fatty acids, lipids etc., and a therapeutically active compound or a mixture of therapeutically active compounds. Similar as shown above, various alternatives are possible when formulating the final liposomal composition.

In a first alternative, a liposomal composition may be formed by reacting in a first step the fatty acid and/or lipid components (portion (II)) etc., with portion (I), thereby obtaining a conjugate component. Subsequently, this conjugate component may be reacted with the therapeutically active compound or a mixture of therapeutically active compounds to obtain the final liposomal composition.

In a second alternative, a liposomal composition may be formed by reacting in a first step a fatty acid and/or lipid components (portion (II)) etc., with a therapeutically active compound or a mixture of therapeutically active compounds, wherein the fatty acid and/or lipid components (portion (II)) etc. have not yet been reacted with portion (I), the cargo moiety. A preliminary liposomal composition obtained thereby includes any commercially available or otherwise readily prepared liposomal composition. In a second step, the fatty acid and/or lipid components (portion (II)) etc., contained in such a preliminary liposomal composition, are reacted with portion (I) as defined herein, resulting in a final liposomal composintion.

Further to the above, a liposomal composition, may be protected against degradation by the reticulo endothelial system by reacting the liposomal composition with a polymer as defined above. Therefore, the polymer is preferably incorporated into the liposomal membrane. The polymers used for obtaining such a "stealth effect" is preferably a conjugate polymer as defined above, i.e. a polymer (portion (II)), which already has been reacted with portion (I), the cargo moiety. Alternatively, the polymer (portion (II)) used therefore, may be reacted with portion (I) similar as disclosed above subsequent to reacting the polymer with the liposomal composition. In both cases a final liposomal composition is obtained comprising, wherein polymer conjugates as defined above are preferably incorporated into the liposomal membrane.

Portion (II) of the inventive conjugate, may also be any of the above cargo moieties modified by micro- and nanoparticles, PLGA particles, supermagnetic particles, magnetic beads, or to semiconductors (quantum dots). Portion (I) then may bind to the cargo moiety as defined above or to the attached micro- and nanoparticles, PLGA particles, supermagnetic particles, magnetic beads, or to semiconductors (quantum dots).

Conjugate molecules of the invention are composed of at least one portion (I) and one portion (II). More precisely, the conjugate molecule according to the invention may comprise 1 to 10, preferably 1 to 25 and more preferably 1 to 50 portions (I) per portion (II). It will be preferred that the number of attached portions (I) rise with the size of portion (II). By way of example conjugate molecules comprising macromolecules as defined above as portion (I), such as antibodies, proteins, polymers, etc., preferably exhibit a larger number of portion (I) (e.g. 1-50) than e.g. small nucleic acid sequences, small organic compounds, etc. (1-10, 1-25).

Preferably, portion (I) and portion (II) are linked by chemical or electrostatic coupling in any suitable manner known in the art. More preferably, portion (I), as defined above by formula (I), is covalently bound to portion (II) of the conjugate molecule. As defined above, molecules representing portions (I) and (II) of the inventive conjugate moiety preferably comprise any chemically reactive moiety which allows to react portion (I) with portion (II) as defined below. Preferably, the chemically reactive moiety of portion (II) is a reactive moiety such as an amine moiety, a sulfhydryl moiety, a hydroxyl moiety, etc.. It may be located at any position of a molecule representing portion (II), preferably at its terminal end or at side chains, e.g. sugar side chains. If portion (II) is e.g. a DNA, the chemically reactive moiety may be located at its 5'- or 3' terminus, or, if portion (II) is a protein, at its N- or C-terminus or at its side chains. If portion (II) is an antibody, the chemically reactive moiety may be located at its N- or C-terminus or at any position accessible at the surface. A covalent bond between portions (I) and (II) may then be obtained by reacting the reactive moiety of a portion (I) with the reactive moiety of a portion (II).

In one alternative, the chemically reactive moiety of portion (I) forms a suitable leaving moiety with a chemically reactive moiety of portion (II), which allows to covalently link portion (I) with portion (II). The leaving moiety is typically removed subsequent to establishing a covalent bond between portions (I) and (II) within a substitution reaction. Preferably, the chemically reactive moiety of portion (I) may be a moiety as defined above, e.g. a N-succinimidyl moiety, a pyridyl moiety, etc., whereas the chemically reactive moiety of portion (II) may be an amine moiety, a sulfhydryl moiety or any other chemically reactive moiety as defined above._More preferably, portion (I) may finally be covalently linked with portion (II) e.g. via an amine bond according to following exemplary reaction scheme (wherein R represents portion (II)):

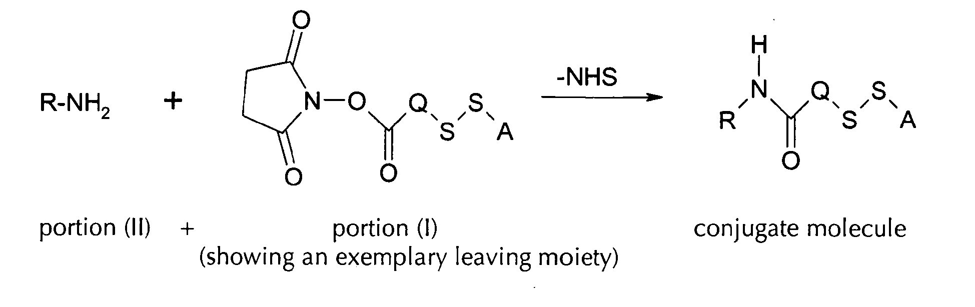

In a specific example, a portion (I) according to formula (I) may be a N-succinimidyl containing molecule derived from SPDP (N-succinimidyl-3-(2pyridyldithio) propionate), Ic-SPDP, Sulfo-Ic-SPDP, etc.. The covalent bond between portion (I) and portion (II) is then preferably formed by reacting SPDP with the amine moiety of portion (II) (represented by R).

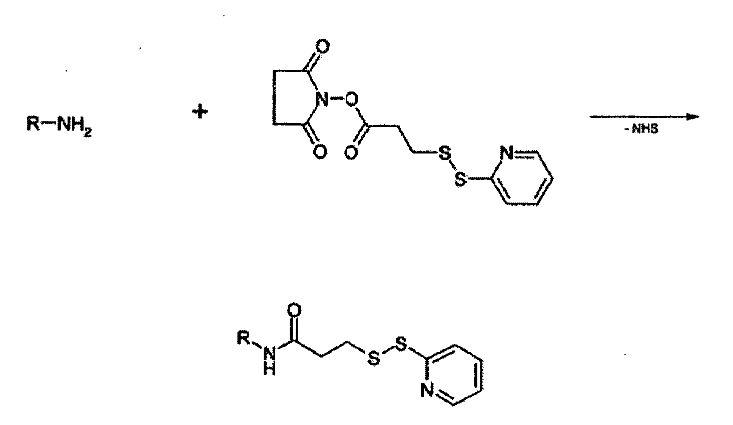

In another specific example, the coupling of portion (I) with portion (II) may be carried out via a sulfhydryl moiety being the reactive moiety of portion (II). The reaction may be performed according to the following exemplary reaction scheme:

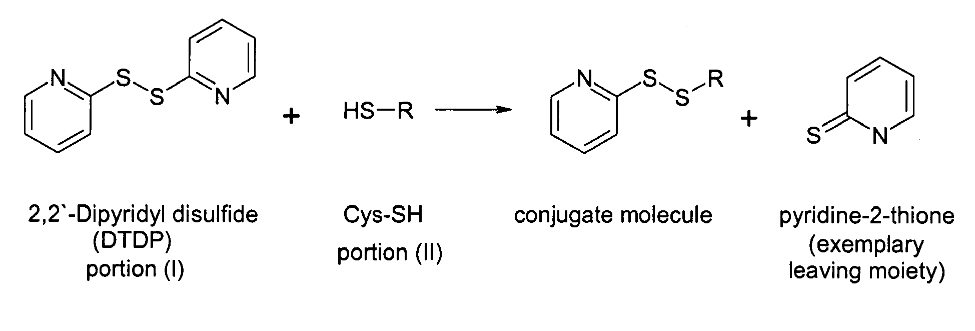

The coupling of portion (I) with portion (II) may also be carried out by an addition reaction e.g. via any of the above mentioned reactive moieties, e.g. a sulfhydryl moiety. Such an addition reaction typically does not require the presence of a leaving moiety and may be carried out according to the following exemplary reaction scheme:

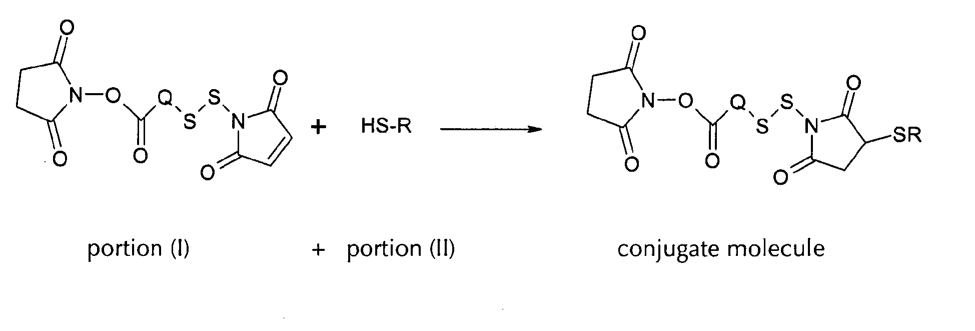

Portion (I) may also be reacted with any suitable reactive moiety of a portion (II) such as a sugar side chain according to the following exemplary reaction scheme:

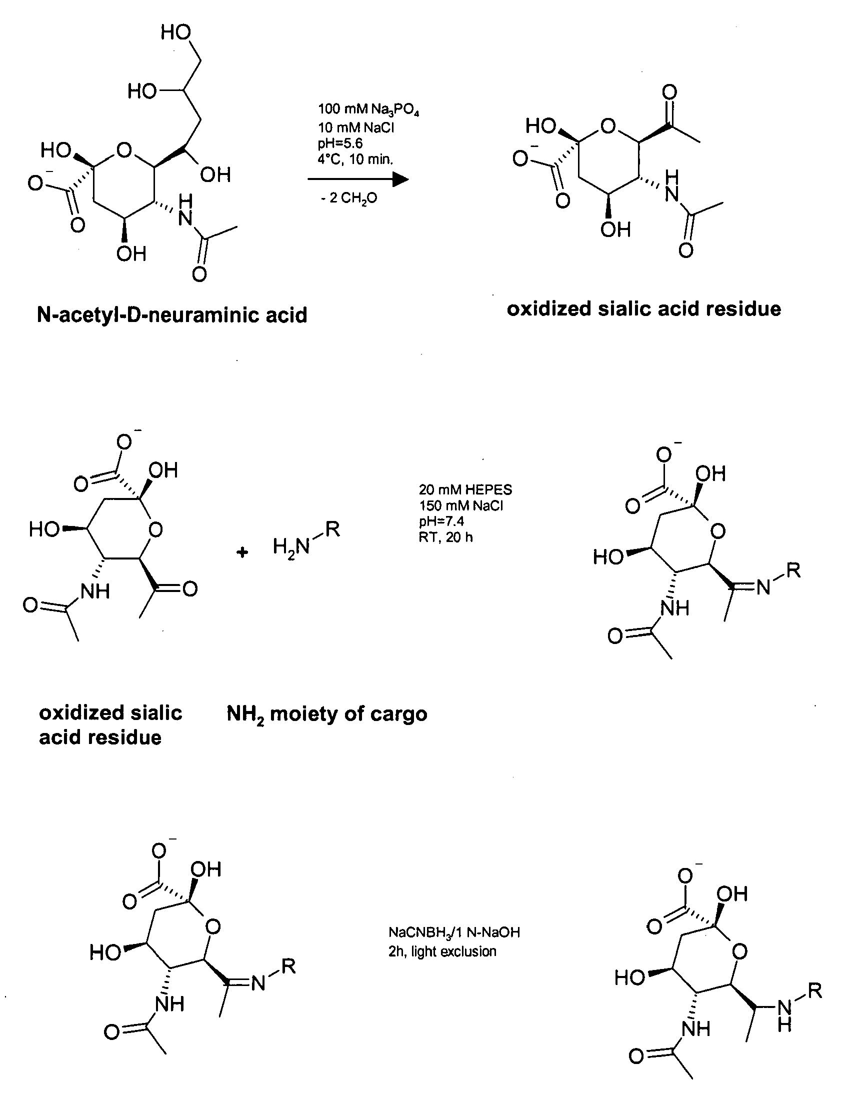

Any of the above coupling reactions above may be carried out in a suitable buffer, such as phosphate buffered saline (PBS) buffer, Tris-buffer, Tris-HCl buffer, etc. The temperature selected for coupling portion (I) with portion (II) preferably lies within a range of about 0 to 40°C, more preferably between 15 to 30°C and most preferably at room temperature (RT), i.e. 20-25°C. Typically, the pH of the coupling reaction lies within a range of from 5 to 10, more preferably within a range of from 6 to 9, and most preferably between 7 and 8. Portion (I) is preferably added to portion (II) in an excess of about 1 to 50fold, more preferably 5 to 10fold of portion (I) over portion (II).

However, attention is drawn to the fact that portion (I) of such an inventive conjugate molecule preferably contains a covalent bond, such as a disulfide, that is cleavable under cellular conditions. Consequently, cargo moieties (portions (II)) as defined above can be transferred into the cell and/or the nucleus, whereby the thiopyridyl moiety (portion (I)) may be cleaved off, thereby releasing portion (II).

Apart from portions (I) and (II), an inventive conjugate molecule may comprise an additional portion (e.g. portion (III)). Preferably, such an additional portion is fused to a portion (II) as defined above, more preferably by a covalent bond. More preferably, such an additional portion (e.g. portion (III)) allows the inventive conjugate molecule to specifically bind to a preselected cell type, e.g immune cells, hepatocytes, in particular tumor cells of organ, e.g. lymphocytes, neurons etc. Specificity is achieved by fusing ligands (e.g. ligands for extracellular portions of membrane proteins, like receptors, or mono- or bispecific antibodies/antibody fragments directed to extracellular portions of membrane proteins, tumor cell markers), which bind to these cell markers. Thereby, the inventive conjugate molecule may be directed specifically to target cells or tissues of an animal to be treated.

Any of the above mentioned portion (II) and or portion (III) of the inventive conjugate may be labelled for detection, e.g. in cell culture assays. In a more preferred embodiment portion (II) or portion (III) of the inventive conjugate may carry at least one label selected from:
(i) radioactive labels, i.e. radioactive phosphorylation or radioactive label with sulphur, hydrogen, carbon, nitrogen, etc.
(ii) coloured dyes (e.g. digoxygenin, etc.)
(iii) fluorescent groups (e.g. fluorescein, etc.)
(iv) chemoluminescent groups,
(v) immobilizing moieties, e.g. HA, HSV-tag, His6-tag, Strep-tag, etc.
(vi) a combination of labels of two or more of the labels mentioned under (i) to (v).

In a preferred embodiment portion (II) or portion (III) of the inventive conjugate molecule may be labelled with non-bulky labels and/or with a label exerting no deleterious effects to a cell. These additional labels can be linked to portion (II) or portion (III) of the inventive conjugate molecule at a suitable position, for example, the N-terminus, the C-terminus of a peptide or protein sequence, 5' or 3' of a nucleotide sequence or internally in these sequences. Such labels (e.g, HA, HSV-Tag, His6) may render the inventive conjugate molecule amenable to purification and/or isolation. If desired, a possible interaction partner can then be detected and isolated by utilizing any of the aforementioned labels.

It should be appreciated, that the invention refers to clinical applications and the inventive conjugate may also be advantageously applied in medical and biological research, e.g. in cell culture assays. In order to make the present invention amenable to clinical use a pharmaceutical composition is provided which comprises a conjugate molecule of the invention as therapeutic molecule and optionally a pharmaceutically acceptable carrier, adjuvant and/or vehicle. This pharmaceutical composition is intended for use in gene therapy purposes, for the treatment, diagnosis or prophylaxis of autoimmune diseases or infectious diseases or for induction of apoptosis in cells, etc.. The pharmaceutical composition further may be used for treatment or prophylaxis of diseases including cancer diseases, neoplastic conditions or tumors, etc..

Without being bound to theory, conjugate molecules as contained in the inventive pharmaceutical compositions are delivered into cells and in particular into the nucleus by using physiologically occurring transport receptors exhibiting free thiol groups on their surface. Thereby, the inventive conjugates may bind to free reactive moieties, e.g. thiol groups presented by the receptor via their portions (I). Being bound by such transporter molecules, transport into cells and particularly into the nucleus utilizing physiological transport mechanisms may be carried out.

The inventive pharmaceutical composition may be used for gene therapy purposes. By utilizing the inventive conjugate in a pharmaceutical composition according to the present invention, preferably pharmaceutically active portions (II) (when bound to portion (I)) readily penetrate cell membranes and, if necessary, nuclear membranes. By providing an efficient way of penetrating nuclear membranes, a major problem of gene therapy is overcome, since up to date, efficiency in gene therapy typically suffers from a non-penetration or insufficient penetration of cellular and/or nuclear membranes. By way of example, e.g. a nucleic acid sequence used as portion (II) as defined above may be carried into the cell and particularly into the nucleus. Such a nucleic acid sequence may contain an altered genomic sequence, which may be integrated into the genome e.g. by homolog recombination mechanisms of the cell, by site-directed (phi)C31 integrase, etc.. Alternatively, the nucleic acid sequence may encode a protein as defined herein and express same when arrived in the cell. It is also possible to deliver cDNA sequences, which may specifically suppress transcription and/or translation of genes. Furthermore, artificial chromosomes, plasmid DNA, oligonucleotides, siRNA, etc. may be delivered by using the inventive pharmaceutical composition.

The inventive pharmaceutical composition may further be used in the treatment of autoimmune diseases. Autoimmune diseases in context of the present invention may be selected e.g. from multiple sclerosis (MS), rheumatoid arthritis, diabetes, diabetes type I, systemic lupus erythematosus (SLE), chronic polyarthritis, Basedow's disease, autoimmune forms of chronic hepatitis, colitis ulcerosa, allergy type I-diseases, allergy type II-diseases, allergy type III-diseases, allergy type IV-diseases, fibromyalgia, alopecia, Morbus Bechterew, Morbus Crohn, Myasthenia gravis, neurodermitis, polymyalgia rheumatica, progressive systemic sclerosis (PSS), psoriasis, Reiter's syndrome, rheumatic arthritis, vaskulitis, etc..

Infectious diseases to be treated with an inventive pharmaceutical composition may also be selected from e.g influenza, malaria, SARS, amarillic typhus (yellow fever), Lyme-Borreliosis, Leischmaniosis, anthrax, meningitis, viral infectious diseases such as AIDS, condyloma acuminata, Dengue fever, tertian fever, Ebola virus, cold, FSME, angina, angina pectoris, hepatitis, Herpes-Simplex type I, Herpes-Simplex Type II, Herpes zoster, influenza, japanese encephalitis, Lassa fever, Marburg virus, measles, aphthous fever, mononucleosis, mumps, Norwalk virus infection, Pfeifer's gland fever, pox, poliomyelitis, pseudo croup, fifth disease, hydrophobia (rabies), warts, west-Nil-fever, varicella, Cytomegalo-Virus (CMV) infection, bacterial infectious diseases such as abort (prostata inflammation), appendicitis, borreliosis, botulisms, campylobacter, Chlamydia trachomatis, cholera, diphtheria, donavanosis, epiglottises, epidemic typhus, typhus fever, gonorrhea, rabbit fever, helicobacter pylori, pertussis, climatic bubo, bone marrow inflammation, legionnaires' disease, Lepra, listeriosis, pneumonia, meningitis, bacterial and abacterial meningitides, otitid media, mycoplasmatic hominis, chorioamnionitis, noma, paratyphus, pest, Reiter's syndrome, Rocky Mountain spotted fever, salmonella paratyphus, salmonella typhus, scarlatina, syphilis, tetanus, gonorrhea (tripper), tsutsugamushi, tuberculosis, vaginitis (colpitis), and infectious diseases caused by parasites, protozoa or fungi such as amebiasis, bilharziosis, chagas syndrome, echinococcus, fish tapeworm, Ciguatera, fox tapeworm, athletes' foot, dog tapeworm, candiosis (thrush), dross (scabies), cutane Leishmaniosis, visceral Leishmaniosis, Giadiasis, bugs, malaria, onchozercosis, fungal diseases, cattle tapeworm, schistosomiasis, sleeping sickness (trypanosomiasis), pig tapeworm, toxoplasmosis, trichomoniasis, etc.;

Moreover, the inventive pharmaceutical composition may be used in the treatment of cancers, preferably by inducing apoptosis in cancer cells. For this purpose inventive conjugates representing a portion (II) as defined above can be selectively designed to target cancer cells, e.g. by using an additional moiety (e.g. portion (III)) as defined above, and avoid thereby so called multidrug-resistancies as observed with state-of-the-art therapeutics in the treatment of cancer. Contrary to these state-of-the-art therapeutics inventive conjugate molecules selectively penetrate target cells and nuclei but not any cell. Cancers in context of the present invention are preferably selected from colon cancer, melanomas, kidney cancer, lymphoma acute myeloid leukemia (AML), acute lymphoid leukemia (ALL), chronic myeloid leukemia (CML), chronic lymphocyte leukemia (CLL), gastrointestinal cancers, lung cancers, gliomas, thyroid cancers, mamma carcinomas, prostate cancers, hepatoma, virus-induced cancers such as Papilloma virus-induced carcinomas (e.g. Cervixcarcinoma), adenocarcinomas, Herpes virus-induced cancers (e.g. Burkitt's Lymphom, EBV-induced B Cell lymphoma), Hepatitis B-induced cancers, HTLV-1 and HTLV-2 induced lymphoma, acusticus neurinoma, cervical cancer, lung cancer, pharyngeal cancer, anal carcinoma, glioblastoma, lymphoma, rectum carcinoma, astrozytoma, brain cancers, stomach cancer, retinoblastoma, basaliom, brain metastases, medullo blastoma, vaginal cancer, pancreatic cancer, testis cancer, melanoma, bladder cancer, Hodgkin syndrome, meningeome, Schneeberger syndrome, bronchial carcinoma, pituitary cancer, mycosis fungoides, gullet cancer, breast cancer, carcinoides, neurinoma, spinalioma, Burkitts lymphoma, laryngeal cancer, thymoma, corpus carcinoma, bone cancer, non-Hodgkin lymphoma, urethra cancer, CUP syndroma, head-cervix cancers, oligodendroglioma, vulva cancer, bowel cancer, colon carcinoma, oesphaguscarcinoma, verruca involvement, small intestine cancers, craniopharyngeoma, ovarial carcinoma, abdominal cancers, ovarian cancer, liver cancer, pancreatic carcinoma, cervical carcinoma, endometrial carcinoma, liver metastases, penis cancer, tongue cancer, gall bladder cancer, leukemia, plasmozytoma, uterus cancer, palpebra cancer, prostate cancer, etc.;
Likewise, the present invention includes a method for treating a condition, e.g. one of the afore-mentioned conditions, comprising the steps of administering a therapeutically effective amount of a conjugate molecule according to the invention.

Another embodiment of the present invention also refers to the use of the inventive conjugate molecules or pharmaceutical compositions containing inventive conjugate molecules as medicament. Inventive conjugate molecules or pharmaceutical compositions containing inventive conjugate molecules may also be used for the treatment or for the preparation of pharmaceutical compositions for gene therapy, for the treatment of autoimmune diseases or infectious diseases, for induction of apoptosis in cells, or for treatment of diseases including cancer diseases, neoplastic conditions or tumors. Likewise, the present invention includes a method for treating a condition, e.g. one of the afore-mentioned conditions, comprising the steps of administering a therapeutically effective amount of a conjugate molecule according to the invention.

A "pharmaceutically acceptable carrier, adjuvant, or vehicle" according to the present invention refers to a non-toxic carrier, adjuvant or vehicle that does not destroy the pharmacological activity of the inventive conjugate molecule as therapeutic molecule with which it is formulated. Pharmaceutically acceptable carriers, adjuvants or vehicles that may be used in the compositions of this invention include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethyl cellulose, polyacrylates, waxes, polyethylene-polyoxypropylene-block polymers, polyethylene glycol and wool fat.

The pharmaceutical composition of the present invention may be administered parentally or non-parentally (e.g. orally, topically, e.g. by applying an aerosole or an ointment onto the skin, etc.).

The term "parenteral" as used herein includes subcutaneous, intravenous, intramuscular, intra-articular, intra-synovial, intraperitoneally, intrasternal, intrathecal, intrahepatic, intralesional and intracranial injection or infusion techniques. If administered parentally, the pharmaceutical compositions are administered preferably subcutaneously or intravenously. Sterile injectable forms of the pharmaceutical compositions of this invention may be aqueous or oleaginous suspension. These suspensions may be formulated according to techniques known in the art using suitable dispersing or wetting agents and suspending agents. The sterile injectable preparation may also be a sterile injectable solution or suspension in a non-toxic parenterally-acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium.

For this purpose, any bland fixed oil may be employed including synthetic mono- or di-glycerides. Fatty acids, such as oleic acid and its glyceride derivatives are useful in the preparation of injectables, as are natural pharmaceutically acceptable oils, such as olive oil or castor oil, especially in their polyoxyethylated versions. These oil solutions or suspensions may also contain a long-chain alcohol diluent or dispersant, such as carboxymethyl cellulose or similar dispersing agents that are commonly used in the formulation of pharmaceutically acceptable dosage forms including emulsions and suspensions. Other commonly used surfactants, such as Tweens, Spans and other emulsifying agents or bioavailability enhancers which are commonly used in the manufacture of pharmaceutically acceptable solid, liquid, or other dosage forms may also be used for the purposes of formulation.

If administered orally, the pharmaceutical compositions of this invention may be administered in any orally acceptable dosage form including, but not limited to, capsules, tablets, aqueous suspensions or solutions. In the case of tablets for oral use, carriers commonly used include lactose and corn starch. Lubricating agents, such as magnesium stearate, are also typically added. For oral administration in a capsule form, useful diluents include lactose and dried cornstarch. When aqueous suspensions are required for oral use, the active compound is combined with emulsifying and suspending agents. If desired, certain sweetening, flavouring or colouring agents may also be added. Additionally, standard pharmaceutical methods can be employed to control the duration of action. These are well known in the art and include control release preparations and can include appropriate macromolecules, for example polymers, polyesters, polyaminoacids, polyvinylpyrrolidone, ethylenevinylacetate, methyl cellulose, caraboxymethyl cellulose or protamine sulfate. The concentration of macromolecules as well as a the methods of incorporation can be adjusted in order to control release. Additionally, the agent can be incorporated into particles of polymeric materials such as polyesters, polyaminoacids, hydrogels, poly (lactic acid) or ethylenevinylacetate copolymers. In addition to being incorporated, these agents can also be used to trap the molecule in microcapsules.

Further administration forms are e.g. by inhalation spray, topically, rectally, nasally, buccally, vaginally or via an implanted reservoir, some of which are described in the following in more detail.

Accordingly, the pharmaceutical compositions of this invention may be administered in the form of suppositories for rectal administration. These can be prepared by mixing the agent with a suitable non-irritating excipient that is solid at room temperature but liquid at rectal temperature and therefore will melt in the rectum to release the drug. Such materials include cocoa butter, beeswax and polyethylene glycols.

The pharmaceutical compositions of this invention may also be administered topically, especially when the target of treatment includes areas or organs readily accessible by topical application, including diseases of the eye, the skin, or the lower intestinal tract. Suitable topical formulations are readily prepared for each of these areas or organs. Topical application for the lower intestinal tract can be effected in a rectal suppository formulation (see above) or in a suitable enema formulation. Topically-transdermal patches may also be used.

For topical applications, the pharmaceutical compositions may be formulated in a suitable ointment containing the active component suspended or dissolved in one or more carriers. Carriers for topical administration of the conjugate molecules of this invention include, but are not limited to, mineral oil, liquid petrolatum, white petrolatum, propylene glycol, polyoxyethylene, polyoxypropylene molecule, emulsifying wax and water. Alternatively, the pharmaceutical compositions can be formulated in a suitable lotion or cream containing the active conjugate molecules suspended or dissolved in one or more pharmaceutically acceptable carriers. Suitable carriers include, but are not limited to, mineral oil, sorbitan monostearate, polysorbate 60, cetyl esters wax, cetearyl alcohol, 2-octyldodecanol, benzyl alcohol and water.

For ophthalmic use, the pharmaceutical compositions may be formulated as micronized suspensions in isotonic, pH adjusted sterile saline, or, preferably, as solutions in isotonic, pH adjusted sterile saline, either with or without a preservative such as benzylalkonium chloride. Alternatively, for ophthalmic uses, the pharmaceutical compositions may be formulated in an ointment such as petrolatum.

The pharmaceutical compositions of this invention may also be administered by nasal aerosol or inhalation. Such pharmaceutical compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other conventional solubilizing or dispersing agents.

Most preferably, the pharmaceutical compositions of this invention are formulated for oral administration.

The amount of the conjugate molecule(s) of the present invention that may be combined with adjuvants and vehicles to produce a pharmaceutical composition in a single dosage form will vary depending upon the host treated, the particular mode of administration. Preferably, the pharmaceutical compositions should be formulated so that a dosage of between 0.01-100 mg/kg body weight/day of the inhibitor can be administered to a patient receiving these compositions. Preferred dosages range from 0.1 - 5 mg/kg body weight/day, even further preferred dosages from 1 - 5 mg/kg body weight/day.

Useful pharmaceutical dosage forms for administration of the conjugates of this invention can be illustrated as follows.

Capsules: Capsules are prepared by filling standard two-piece hard gelatin capsulates each with 100 milligram of powdered active compound, 175 milligrams of lactose, 24 milligrams of talc and 6 milligrams magnesium stearate. Soft Gelatin Capsules: A mixture of active compound in soybean oil is prepared and injected by means of a positive displacement pump into gelatin to form soft gelatin capsules containing 100 milligrams of the active compound. The capsules are then washed and dried. Tablets: Tablets are prepared by conventional procedures so that the dosage unit is 100 milligrams of active compound. 0.2 milligrams of colloidal silicon dioxide, 5 milligrams of magnesium stearate, 275 milligrams of microcrystalline cellulose, 11 milligrams of cornstarch and 98.8 milligrams of lactose. Appropriate coatings may be applied to increase palatability or to delay absorption. Injectable: A parenteral composition suitable for administration by injection is prepared by stirring 1.5% by weight of active compounds in 10% by volume propylene glycol and water. The solution is made isotonic with sodium chloride and sterilized. Suspension: An aqueous suspension is prepared for oral administration so that each 5 millimeters contain 100 milligrams of finely divided active compound, 200 milligrams of sodium carboxymethyl cellulose, 5 milligrams of sodium benzoate, 1.0 grams of sorbitol solution U.S.P. and 0.025 millimeters of vanillin.

It has to be noted that a specific dosage and treatment regimen for any particular patient will depend upon a variety of factors, including the activity of the specific conjugate molecule employed, the age, body weight, general health, sex, diet, time of administration, rate of excretion, drug combination, and the judgment of the treating physician and the severity of the particular disease being treated. The amount of conjugate molecules of the present invention in the pharmaceutical composition will also depend upon the particular conjugate molecule in the composition.

The terms used herein shall be interpreted in the following way. The term "therapeutic" as used here, for example, in the terms "therapeutic molecule" and "therapeutically effective amount" means to have at least some minimal physiological effect. For example, a "therapeutic molecule" would have at least some minimal physiological effect upon being administered to a living body. An agent may have at least some minimal physiological effect upon administration to a living body if, for example, its presence results in a change in the physiology of a recipient animal. For example, a physiological effect upon administering a "therapeutic" anti-tumor molecule may be the inhibition of tumor growth, or decrease in tumor size, or prevention reoccurrence of the tumor. Administration of a "therapeutically effective amount" means the amount administered is physiologically significant. An agent is physiologically significant if its presence results in a change in the physiology of a recipient animal. For example, in the treatment of cancer or neoplastic disease, a molecule which inhibits the growth of a tumor or decreased the size of the tumor or prevents the reoccurrence of the tumor would be considered therapeutically effective. The term "anti-tumor drug" means any therapeutic agent having therapeutic effect against a tumor, neoplastic disease or cancer. The term "drug" means any agent having a therapeutic effect when administered to an animal. The dosage of the present administration for therapeutic treatment will be sufficient to generate a therapeutically effective amount of the administered agent. The term "condition" means any condition, state, disease, abnormality, imbalance, malady and the like in an animal which one seeks to effect by administrating a therapeutically effective amount of a therapeutic molecule. The term "treating", used for example in the term "treating a condition", means at least the administration of a therapeutically effective amount of a therapeutic molecule to elicit a therapeutic effect. It does not necessarily imply "curing", but rather having at least some minimal physiological effect upon a condition upon administration to a living body having a condition. For example, treatment could encompass administering an agent and the presence of that agent resulting in a change in the physiology of a recipient animal.

### Description of Figures:

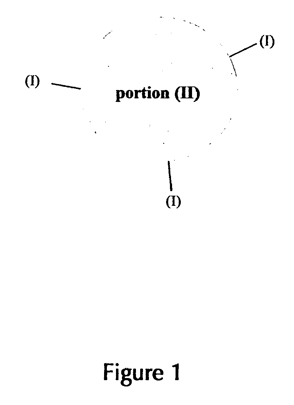

- **Figure 1**:: shows a schematic diagram of the inventive conjugate molecule. The cargo moiety to be transported (portion (II)), e.g. a protein such as BSA, is modified with at least one thiopyridyl moiety (portion (I), here represented by "(I)"). The conjugation rate is typically between 1 to 50.

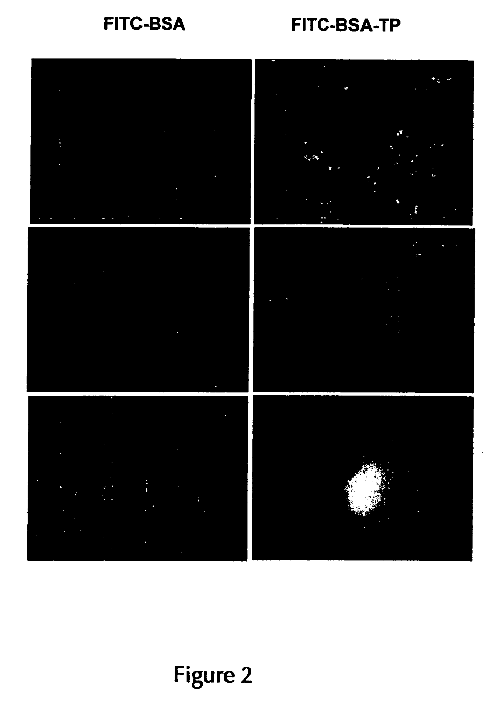

- **Figure 2**:: shows an epifluorescence photograph of the cellular and nuclear uptake of thiopyridyl (TP) modified FITC-BSA. HepG2 cells were incubated with FITC-labelled BSA conjugates and fixed in paraformaldehyde. The cellular and nuclear epifluorescence photographs show an FITC signal of BSA in the left column and an FITC-BSA-TP signal in the right column. Uptake of FITC-BSA-TP into the nucleus is clearly observable in the right column. The corresponding nucleoli were colored using the fluorescent dye propidiumiodid.

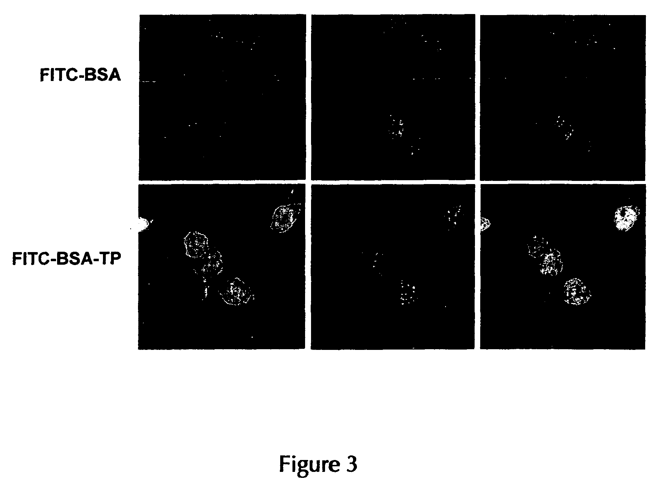

- **Figure 3:**: shows an confocal laser scanning photograph of the cellular and nuclear uptake of thiopyridyl (TP) modified FITC-BSA. HepG2 cells were incubated with FITC-labelled BSA conjugates and fixed in paraformaldehyde. The cellular and nuclear confocal laser scanning photographs show an FITC signal of BSA in the upper row and an FITC-BSA-TP signal in the bottom row. Uptake of BSA into the nucleus is clearly observable in the right column of the bottom row. The corresponding nucleoli were colored using the fluorescent dye propidiumiodid and are shown in the middle column. The right column represents an overlay of the first and the second column.

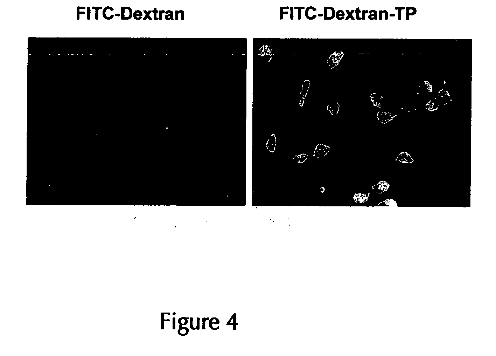

- **Figure 4**:: shows an epifluorescence photograph of the cellular and nuclear uptake of thiopyridyl (TP) modified FITC-Dextran. HepG2 cells were incubated with FITC-labelled Dextran conjugates and fixed in paraformaldehyde. The cellular and nuclear epifluorescence photographs show an FITC signal of Dextran (left photograph) and an FITC-Dextran-TP signal (right photograph). Uptake and coloring of the nucleoli are as described above for Figure 2.

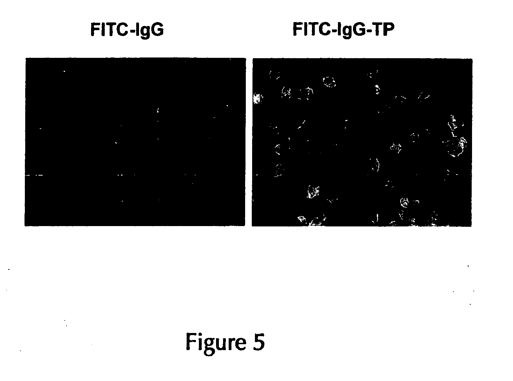

- **Figure 5:**: shows an epifluorescence photograph of the cellular and nuclear uptake of thiopyridyl (TP) modified FITC-IgG. HepG2 cells were incubated with FITC-labelled IgG conjugates and fixed in paraformaldehyde. The cellular and nuclear epifluorescence photographs show an FITC signal of IgG (left photograph) and an FITC-IgG-TP signal (right photograph). Uptake and coloring of the nucleoli are as described above for Figure 2..

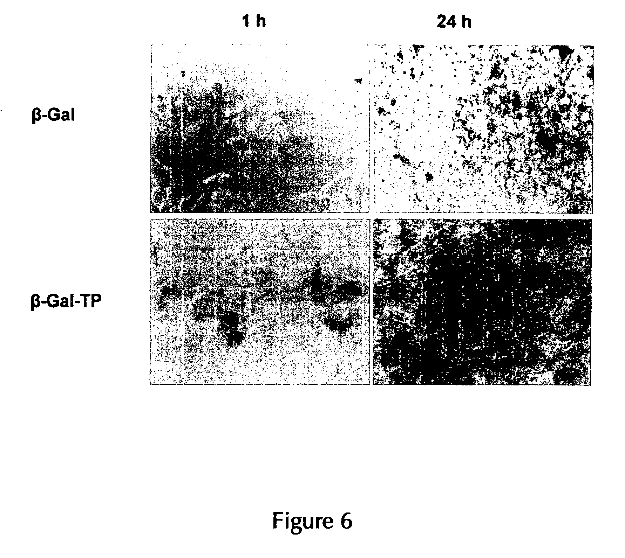

- **Figure 6**:: shows a photograph of the cellular and nuclear uptake of thiopyridyl (TP) modified β-galactosidase. Therefore, HepG2 cells were incubated with (TP) modified β-galactosidase and fixed in paraformaldehyde. β-galactosidase activity was visualized by using X-Gal. Uptake of β-galactosidase into the nucleus after 1 and 24h is clearly observable in the bottom row versus the control (top row).

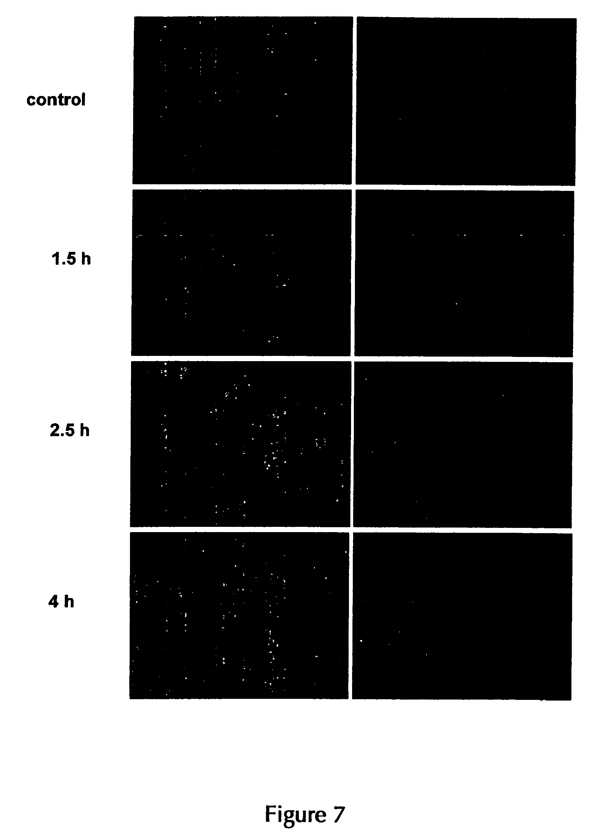

- **Figure 7**:: shows an epifluorescence photograph of the cellular and nuclear uptake of thiopyridyl (TP) modified PEG-liposomes. Therefore, HepG2 cells were incubated with thiopyridyl (TP) modified PEG-liposomes and fixed in paraformaldehyde. The nucleoli were colored with DAPI. From top to bottom a control experiment and the results from 1 1.5, 2.5 and 4h incubation are shown. After 1.5 h incubation of cells with liposomes colored endocytotic vesicles are observable. After 2.5 h incubation many signals are observable being associated with the nucleus. After 4 h incubation the fluorescence signal can be observed in the vesicular structures of the cells and as an diffuse signal in the cytoplasm indicating release from the endosomal compartment.

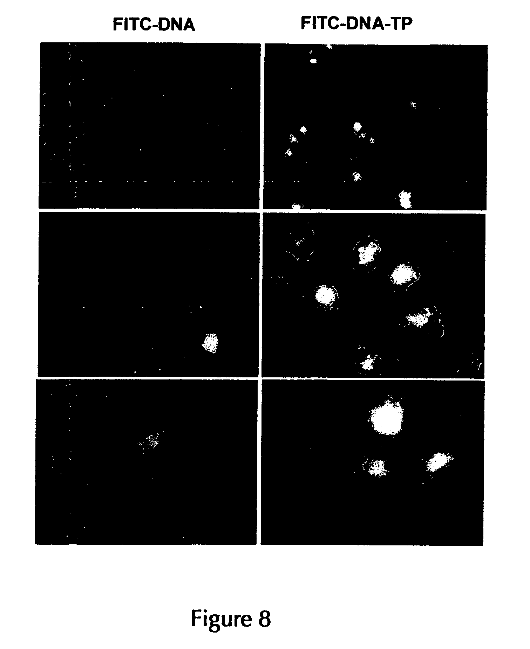

- **Figure 8**:: shows an epifluorescence photograph of the cellular and nuclear uptake of thiopyridyl (TP) modified FITC-plasmids Therefore, HepG2 cells were incubated with FITC-labelled plasmid conjugates and fixed in paraformaldehyde. The cellular and nuclear epifluorescence photographs show an FITC signal of plasmids in the left column and an FITC-plasmid-TP signal in the right column. Uptake of plasmid-DNA into the nucleus is clearly observable in the right column. The corresponding nucleoli were colored using the fluorescent dye propidiumiodid. Uptake of plasmid-DNA is clearly observable in the right column.

The following figures and examples are thought to illustrate the invention and should not be constructed to limit the scope of the invention thereon. All references cited by the disclosure of the present application are hereby incorporated in their entirety by reference.

### Examples:

### 1. Materials and methods

### 1.1 Fluorescence labeling of BSA with carboxyfluorescein-N-hydroxysuccinimidester

100 mg BSA (1.59 µmol, Roche, #775860) were solubilized in 2.0 ml PBS (pH = 8) and reacted with a 5-fold excess of 5(6)-carboxyfluorescein-N-hydroxysiccinimidester (7.95 µmol, Aldrich, #21878) with exclusion from light and agitating for 1 h. Separation from unbound FITC was carried out on a PD-10 column (Amersham, Freiburg, Germany, #17-0851-1). Determination of the degree of FITC-conjugation (~1) was carried out photometrically (BSA ε₂₇₈ = 39,000/Mcm, FITCε₄₉₈ = 82,500/Mcm). In an analogous manner coupling of FITC to aminodextran (Molecular Probes, 21.5 mol amine/mol) was carried out, as well as coupling to IgG1 (Bovine IgG, whole molecule, Rockland, Gilbertsville, PA, USA, Cat #0010102). In a further step conjugates were purified using a Sephadex G-25 superfine HR 10/10 column (Pharmacia, Uppsala, Sweden).

### 1.2 Coupling of SPDP to FITC-labelled conjugates

80 mg FITC-BSA (1.22 µmol in 5 ml PBS (pH = 8) were reacted with a 10-fold excess of 3-(2-pyridyldithio) propionic acid-N-hydroxy-succinimidester (SPDP) at room temperature with exclusion from light for 1 h and purified subsequently by chromatography (Sephadex G-25 superfine HR 10/10 column; Pharmacia; Uppsala, Sweden) in PBS (pH = 8). FITC-BSA content was determined photometrically (BSA ε₂₇₈ = 39,500/Mcm). The degree of PDP-conjugation was determined subsequent to cleaving off thiopyridone by adding β-mercaptoethanol at 343 nm (ε = 8080 M/cm). The degree of conjugation was determined to be n(PDP)/n(BSA) ~ 9-10. FITC-dextran, FITC-IgG and β-Galactosidase were reacted in an analogous manner. However, when conjugating β-Galactosidase just 8 equivalents SPDP were added and reaction was carried out at room temperature over night. The degree of conjugation was determined to be about 1. When conjugating FITC-IgG just 8 equivalents SPDP were added. Here, the resulting degree of conjugation was determined to be about 3. PBS saturated with helium was used for the conjugations above.

### 1.3 Modification of FITC-BSA-TP conjugates

FITC-BSA was reacted with a 1 0-fold excess of cystein in PBS (pH = 8, He-saturated) for 3 h. Photometric determination of cleaved thiopyridone indicated complete reaction of FITC-BSA-TP to FITC-BSA-Cys. The conjugate was purified chromatographically (Sephadex G-25 superfine HR 10/10 column; Pharmacia, Uppsala, Sweden). FITC-BSA-TP was reacted in presence of 13 mM DTT for 2 h and purfied by chromatography in order to obtain a FITC-BSA conjugate with free thiol-function. Determination of degree of conjugation with Ellmanns' reagent indicated complete separation of the thiopyridone moiety, which, in consequence, led to FITC-BSA-SH. The synthesized FITC-BSA-SH conjugate was further reacted with a 50-fold excess of N-ethylmaleimide for 2 h and purified by chromatography subsequently. Controlling the product for remaining thiol moieties by using Elmanns' reagent indicated a complete reaction to FITC-BSA-NEM.

### 1.4 Coupling of FITC and SPDP to plasmid-DNA

Coupling of FITC and SPDP with plasmid-DNA was carried out by photochemical coupling using psoralenamin. Therefore, a kit from company Sima was used (PS-amine). Coupling was carried out following the manufacturers' instructions. 4 µl of reconstituted psoralenamine solution (4.83 psoralenamine) were pipetted to 100 µl solubilized DNA (pCLuc in pur. water, 200 µg/ml DNA). The resulting solution was vortexed vigorously. The solution was irradiated with UV (λ = 366nm, 6 watt, 850 µwatt/cm²) for 45 minutes on ice. Portions of each 50 µl were purified using a G-25 Microspin column (Amersham) and, subsequently, 2 µl 10xPBS were added. To one 50µl fraction of the purified Psoralenamin-coupled DNA 2 µl FITC-NHS solution (10 µg/ml in DMF) were added. To the other 50µl fraction a mixture consisting of 2 µl FITC-NHS (10 mg/ml in DMF) 2 µl SPDP (10 mg/ml in EtOH) were added. The solutions were agitated for 1.5 h at room temperature. Subsequent thereto plasmid-DNA conjugates were purified repeatedly using a G-25 Microspin column (Amersham), and used for cell culture experiments.

### 1.5 Uptake of conjugates into cells

Corresponding cell lines were seeded in 8-chamber sample carriers (Falcon, #4108, BD Bioscience, Belgium) resulting in about 70-80% confluence next day. Cells were incubated along with inventive conjugates for 1 h at 37°C and 5% CO₂ in an incubator (c = 5 µM). Subsequently thereto cells were washed twice with PBS and fixed with a solution containing 4% paraformaldehyde. The nucleoli were colored by using the fluorescence dye DAPI (0.3 µM) or propidiumiodid (3 µM) and cells were embedded (Vectashield, Vector Laboratories, Burlingame, CA, USA). Photographs were taken using a Sony 3CCD camera having an Axiovert 135-microscope (Carl-Zeiss, Göttingen, Germany; objective: Plan-Neofluar 100x/1,3 oil). Optical transsections for laser scanning microscopy were performed using a confocal microscope having three channels (TCS 4D; Leica Inc. Deerfield, IL) equipped with a Plan Apo 63x/1.32 oil objective). Both fluorescence dyes FITC and propidiumiodide were irradiated at wavelengths 488nm and 567nm using an argon/krypton laser. 256x256 8 bit pictures were generated having an axial distance of 250 nm and a pixel size of 1 nm. Each picture series consisted of about 20-25 pictures. Both fluorochromes were mapped sequentially in the same nucleolus layer.

### 1.6 Nuclear import assay with digitonin permeabilized cells

Corresponding cell lines were seeded in 8-chamber sample carriers (Falcon, #4108, BD Bioscience, Belgium) resulting in about 70% confluence next day. Cells were digitonized (c(Digitonine) = 40 µl/ml in transport buffer; 20 mM HEPES, 110 mM potassium acetate, 5 mM sodium acetate, 2 mM magnesium acetate, 1 mM EGTA, 2 mM dithiothreitol, 1 µg/ml aprotoinine, 1 µg/ml leupeptine, 1 µg/ml pepstatine) on ice for 2 minutes and washed subsequently with transport buffer void of digitonine. FITC-labelled conjugates were diluted with a 1:1 mixture consisting of transport buffer (additional 5 mM creatinine phosphate, 1 mM ATP, 20 u/ml creatinine phosphokinase) and a lysate from reticulocytes (L415/1, Promega, Mannheim, Germany) to a concentration of 5 µM. 125 µl were pipetted into each chamber and incubated for 30 minutes at 37°C (100% air humidity, 5%CO₂). Subsequently, cells were washed twice with PBS and fixed with a solution containing 4% paraformaldehyde. The nucleoli were colored by using the fluorescence dye DAPI (0.3 µM) or propidiumiodid (3 µM) and cells were embedded (Vectashield, Vector Laboratories, Burlingame, CA, USA). Photographs were taken using a Sony 3CCD camera having an Axiovert 135-microscope (Carl-Zeiss, Göttingen, Germany; objective: Plan-Neofluar 100x/1,3 oil).

### 2. Cellular uptake of thiopyridyl (TP)-constructs and transport into the nucleus:

### 2.1 Uptake of BSA

Experiments were carried out for cellular uptake of thiopyridyl (TP)-modified FITC-BSA constructs and transport of these FITC-BSA-TP conjugates into the nucleus. Therefore, HepG2 cells were incubated for 1 h with FITC-labelled BSA conjugates (5 µm) and fixed with paraformaldehyde. Results were analysed by epifluorescence microscopy (see Figure 2). The cellular and nuclear epifluorescence photographs show an FITC signal of BSA in the left column and an FITC-BSA-TP signal in the right column. The corresponding nucleoli were colored using the fluorescent dye propidiumiodid. As a result, incubation of FITC-BSA-TP conjugates for 1 h led to uptake of FITC-BSA-TP constructs into cytoplasm and nucleus of HepG2 cells. Uptake of non-modified FITC-BSA into cytoplasm of HepG2 cells could also be observed. In contrast, no uptake of FITC-BSA into the nucleus could be observed indicating that TP-modification is essential for nuclear uptake of proteins such as BSA.

Confocal laser scanning microscopy was additionally carried out (see Figure 3), since in epifluorescence microscopy the resulting signal assumable may be due to association of the FITC-BSA-TP conjugates with the nuclear membrane. Confocal laser scanning microscopy clearly shows that apart cellular uptake (see Fig. 3., first column, bottom) nuclear uptake of FITC-BSA-TP is observable, thereby confirming results from epifluorescence analysis. Furthermore, apart from localization in the cytoplasm also no localization of non-modified FITC-BSA in the nucleus could be observed using confocal laser scanning microscopy.

### 2.2 Uptake of dextran

Dextran constructs and modifications were prepared analogously to BSA constructs as shown above in section 2.1. As a starting material an amino-modified dextran was used having a molecular weight of 70,000 (MW). Then, TP-modified dextran constructs (5 µm) were incubated with HepG2 cells for 1 h and fixed with paraformaldehyde, which led to cellular and nuclear uptake of FITC-Dextran-TP into cytoplasm and nucleus. Analysis was carried out using epifluorescence microscopy (see Fig. 4). The corresponding nucleoli were colored using the fluorescent dye propidiumiodid. As shown in Figure 4, cellular uptake and nuclear epifluorescence photographs show an FITC signal of Dextran (left picture) and an FITC-Dextran-TP signal (right picture). As can be obtained from the right picture, TP modified FITC-Dextran was taken up by the nucleoli.

Uptake of non-modified FITC-Dextran into cytoplasm of HepG2 cells could also be observed. In contrast, no uptake of FITC-Dextran into the nucleus could be observed indicating that TP-modification is essential for nuclear uptake of dextran molecules.

### 2.3 Uptake of immunglobuline IgG1

Similar as shown in 2.1 and 2.2 IgG1 constructs were analyzed as to whether they may be transported into the cells upon TP modification. IgG1 constructs and modifications were prepared analogously to BSA and dextran constructs as shown above in sections 2.1 and 2.2. As a starting material bovine IgG was used having a molecular weight of approximately 130,000 (MW). Then, TP-modified IgG1 constructs were incubated with HepG2 cells for 1 h and fixed with paraformaldehyde, which led to cellular and nuclear uptake of FITC-Dextran-TP into the cytoplasm and the nucleus of many cells. Analysis was carried out using epifluorescence microscopy (see Fig. 5). The corresponding nucleoli were colored using the fluorescent dye propidiumiodid. As shown in Figure 5, cellular uptake and nuclear epifluorescence photographs show an FITC signal of IgG (left photograph) and an FITC-IgG-TP signal (right photograph). As can be obtained from the right column, TP modified FITC-IgG was taken up by the nucleoli.

Uptake of non-modified FITC-IgG into cytoplasm of HepG2 cells could also be observed. In contrast, no uptake of FITC-IgG into the nucleus could be observed indicating that TP-modification is also essential for nuclear uptake of IgG molecules or related molecules like antibodies.

### 2.4 Uptake of β-galactosidase

Analogous to sections 2.1 to 2.3 above the enzyme β-galactosidase was modified with TP. TP-modification does not exhibit any influence on activity of β-galactosidase. Subsequent to modification HepG2 cells were incubated with (TP) modified β-galactosidase (5 µM) for 1 h or 24 h and then fixed in paraformaldehyde. β-galactosidase activity was visualized by using X-Gal (see Figure 6). As can be observed from Figure 6, incubation of FITC-β-galactosidase-TP with HepG2 cells led to uptake of β-galactosidase into cytoplasm and nucleus into many cells. Along with increased incubation times the number of positive cells rises significantly (see right column, bottom). Subsequent to 24 h incubation almost all cells and their nucleoli showed a positive signal. Only a negligible cellular but no nuclear uptake of non-modified β-galactosidase is observable (see top row, left and right pictures).

### 2.5 Uptake of "stealth-liposomes"

Experiments for cellular uptake of thiopyridyl (TP)-modified PEG based negatively charged liposomes and transport of these conjugates into the nucleus were carried out. PEG based negatively charged liposomes were used showing distal unmodified (carboxylic moiety) or TP-modified PEG chains. The liposomes were modified with fluorescence labelled lipids. Thereafter, HepG2 cells were coincubated with fluorescence labelled liposomes and fixed at different times. In order to detect uptake by cells and nucleus, epifluorescence microscopy was carried out (see Figure 7). The nucleoli were colored with DAPI. From top to bottom a control experiment and the results from 1, 1.5, 2.5 and 4h incubation are shown. As can be obtained from the epifluorescence photographs uptake of liposomes into the nucleus is clearly observable in the right column (Figure 7) in contrast to the left column (Figure 7), showing non-modified PEG-liposomes only. Even after 1.5 h incubation cells with TP-modified liposomes showed colored endocytotic vesicles. After 2.5 h a significant increase in signal intensity was observable associated with uptake of TP-modified liposomes into the nucleus. After 4 h incubation the fluorescence signal was observable as a signal in the vesicular structures of the cell as well as a diffuse signal in the cytoplasm, indicating release of TP-modified liposomes from the endosomal compartment.

### 2.6 Uptake of plasmid-DNA

Similar as demonstrated for BSA, Dextran, IgG and liposomes, experiments for uptake of thiopyridyl (TP)-modified DNA into cytoplasm and nucleus were carried out. Therefore, a reporter plasmid encoding the luciferase gene and having a size of 6.4 kb was TP modified and incubated with HepG2 cells for 1 h. Such incubation led to uptake of TP-modified FITC-DNA into cytoplasm and nucleoli of a significantly large number of cells. Uptake of unmodified FITC-DNA into the cytoplasm was also obserbed. However, no uptake of unmodified FITC-DNA into the nucleus was observed, indicating that TP-modification is essential for nuclear uptake of DNA molecules, e.g. plasmid-DNA, genomic DNA etc..

### 3. Determination of dependency of cellular and nuclear uptake from TP using chemical modification of the TP-substituent with intact cells

In order to determine the impact of the TP-substituent on cellular and nuclear transport the TP substituent of the FITC-BSA-TP conjugate was converted into a free sulfhydryl moiety with dithiothreitol and further reacted with N-ethylmaleimide. Reaction with N-ethylmaleimide blocks the free sulhydryl moiety by chemical coupling. Additionally, FITC-BSA-TP was reacted with the amino acid cystein, which was coupled to FITC-BSA via a disulfide bond. As a result cellular and nuclear uptake are only observable if an intact TP-substituent is present. Neither the free sulfhydryl moiety nor the sulfhydryl moiety blocked by N-ethylmaleimide mediated cellular or nuclear uptake. The same was observed for cystein modified FITC-BSA.

### 4. Determination of dependency of cellular and nuclear uptake from TP using non-fixed and lysed cells

In order to exclude artifacts resulting from fixing of cells FITC-modified BSA and FITC-BSA-TP conjugates (5 µM) were incubated for 1 h with HepG2 cells. Subsequently, cells were immediately transferred in PBS or lysed with lysis buffer within 30 minutes and analyzed via epifluorescence microscopy. The distribution of FITC-BSA-TP conjugates was shown to be identical in both fixed and non-fixed HepG2 cells. Consequently, artifacts can be excluded due to fixing of cells. Additionally, cytoplasma membrane of HepG2 of cells was lysed with lysis buffer (20 mM HEPES, 10 mM KCI, 0.5 mM EDTA, 0.1% Triton X-100, pH7.5) subsequent to incubation. Cell lysis exhibited no influence on cellular distribution of the FITC-BSA-TP conjugate. Even subsequent to cell lysis a fluorescence signal was clearly observed in the nucleus. Consequently, FITC-BSA-TP conjugates are localized in the nucleus.

### 5. Quantification of cellular and nuclear uptake of non-modified and TP-modified FITC constructs using flow cytometrie (FACS)

Exact quantification is a prerequisite for determining efficiency of TP-mediated cellular and nuclear uptake. Cellular and nuclear uptake was thus quantified in flow cytometry experiments using FACS analysis. Therefore, HepG2 cells were incubated for 1 h with FITC labelled conctructs or with FITC labelled and TP-modified conjugates as used in experiments 1 to 4. After incubation cells were detached from the bottom of the cell culture plates (whole cells) by using either a trypsine containing solution or a trypsine containing solution supplemented with detergent Nonidet P 40. Detergent Nonidet P 40 leads to lysis of cytoplasm membranes and release of nucleoli. Propidiumiodid was added to trypsine buffer in order to identify the nucleoli.

As a result, the TP-substituent increases significantly cellular uptake of FITC modified constructs as used in Experiments 1 to 4 (e.g. FITC-BSA (55%), FITC-BSA-PDP (78%), FITC-Dex (25%), FITC-Dex-PDP (55%), FITC-IgG (87%), FITC-IgG-PDP (91%)). Quantitative FACS-analysis of nuclear uptake of TP-modified FITC conjugates subsequent to cell lysis revealed a significant increase in nucleus associated FITC fluorescence (e.g. FITC-BSA (51 %), FITC-BSA-PDP (63%), FITC-IgG (52%), FITC-IgG-PDP (64%), FITC-Dex (7%), FITC-Dex-PDP (84%)).

### Advantages of the present invention

The inventive conjugate molecules allow to improve or even establish cellular and/or nuclear uptake of various types of cargo moieties into cells and/or the nucleus, which could not be transported into the cell and/or the nucleus with state-of-the-art methods.

This transport is accomplished by portion (I) of the inventive conjugate, which comprises a disulfide moiety and allows a cargo moiety (portion (II)) to penetrate the cellular and/or the nuclear membrane. Portion (II) of the inventive conjugate molecule is a therapeutic active molecule preferably selected from proteins, e.g. apoptosis inducing or apoptosis related factors or antibodies, nucleic acids, therapeutically active organic or inorganic molecules, (complex) compositions comprising therapeutically active organic or inorganic molecules, liposomal compositions, polymers, drug carriers, excipients. Consequently, the present inventive conjugate is capable of transporting nearly any therapeutically active molecule or composition into cells and, if desired, into the nucleus. Furthermore, inventive conjugates can be specifically targeted to certain cell types, which allows a skilled person to directly modulate or attack cells, e.g. when treating cancer, infectious diseases, etc.

Finally, the present inventive conjugate provides a completely new molecule class for use in nuclear environment. Up to date no antibody was shown to penetrate the nuclear membrane. Since upon utilizing the inventive conjugates even antibodies may be carried into the nucleus, structures within the nucleus may be targeted now by antibodies, which was not yet possible.

## Claims

1. A conjugate molecule comprising at least one first portion (I) comprising a disulfid containing moiety as a carrier moiety and a second portion (II) as a cargo moiety comprising a therapeutic active molecule selected from proteins, e.g. apoptosis inducing or apoptosis related factors or antibodies, nucleic acids, therapeutically active organic or inorganic molecules, (complex) compositions comprising therapeutically active organic or inorganic molecules, liposomal compositions, polymers, drug carriers, excipients.

2. The conjugate molecule of claim 1, wherein portion (I) is a disulfide containing moiety as defined by general formula (I): 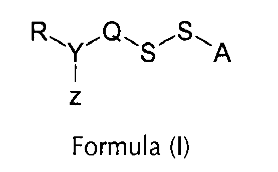 wherein:
R is portion (II), if covalently bound to portion (I), or a (substituted or non substituted) pyridyl moiety, N-maleimido moiety, N-succinimidyl moiety, (C₆H₃)(OH)N₃; C(N₂)CX₃; NHNH₂Cl; or A as defined below, or not present;
Q is selected from the group consisting of:
a bond or (CH₂)ₙ, (CH₂)ₙC(O)NH(CH₂)ₙ, (CH₂)ₙC(O)NHNH₂-HCl, (CH₂)ₙC(O)NH(CH₂)ₙNH, (CH₂)ₙNHC(O)(CH₂)ₙ, (C₆H₄)CH(CH₃), (CH₂)ₙ(C₆H₄), (C₆H₈)CH, (CH₂)ₙNHC(O)(C₆H₄)CH(CH₃), or a or a branched or linear C₁-C₁₀-(hetero)alkyl, C₁-C₁₀-(hetero)alkenyl, C₁-C₁₀-
(hetero)cycloalkyl, C₁-C₁₀-(hetero)cycloalkenyl, C₁-C₁₀-alkoxy, C₁-C₁₀₋alkenoxy, C₁-C₁₀-acyl moiety or a C₁-C₁₀-cycloalkyl moiety, substituted or non substituted C₁-C₁₄-aryl, C₁-C₁₄-heteroaryl, arylalkyl, arylalkenyl, C₅₋₁₄-aryloxy, heteroarylalkyl, heteroarylalkenyl, heterocycloalkyl, heterocycloalkenyl, or heteroaryloxy moiety;
Y is a bond; or C, CH, C-O-, -O-C-, or A as defined below;
Z is H, O, OH, OCH₃, COOH, COOCₙH₂ₙ₊₁, CO, CH₃, (CH₂)ₙCH₃, (CH₂)ₙOH, NH₂, NO, NO₂, NOH, N, NHNH₂, NC, CN, S, SH SO₂; or Z is not present;
A is selected from the group consisting of
CH₂X, or a (substituted or non substituted) C₁-C₁₄-aryl, C₁-C₁₄₋heteroaryl, arylalkyl, arylalkenyl, C₅₋₁₄-aryloxy, heteroarylalkyl, heteroarylalkenyl, heterocycloalkyl, heterocycloalkenyl, or heteroaryloxy moiety, particularly a (substituted or non substituted) pyridyl moiety, N-maleimido moiety, or a N-succinimidyl moiety;
wherein n = 0-10, and X = F, Cl, Br, I, CN or CO.

3. The conjugate molecule of claim 1, wherein portion (I) is a moiety as defined by general formula (II): 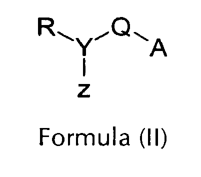 wherein, A, Q, R, Y and Z are as defined above.

4. The conjugate molecule of claims 1 to 3, wherein the at least one first portion (I) and the second portion (II) are linked by a covalent bond.

5. The conjugate molecule of any of claims 1 to 4, wherein portion (II) is a molecule selected from an apoptose inducing or apoptosis related factor selected from or associated with IFF, Apaf, e.g. Apaf-1, Apaf-2, Apaf-3, or APO-2(L), APO-3(L), Apopain, Bad, Bak, Bax, Bcl-2, Bcl-X_{L}, Bcl-xₛ,bik, CAD, calpains, caspases, e.g. caspase-1, caspase-2, caspase-3, caspase-4, caspase-5, caspase-6, caspase-7, caspase-8, caspase-9, caspase-10, caspase-11, granzyme B, or ced-3, ced-9, ceramide, c-Jun, c-Myc, CPP32, crm A, cytochrome C, D4-GDP-DI, Daxx, DcR1, DD, DED, DISC, DNA-PK_{CS}, DR3, DR4, DR5, FADD/MORT1, FAK, Fas, Fas-ligand CD 95/fas (receptor), FLIP, Fodrin, fos, G-Actin, Gas-2, gelsoline, glucocorticoid/ glucocorticoid receptor, granzyme A/B, hnRNPs C1/C2, ICAD, ICE, JNK, Lamin A/B, MAP, MCL-1, Mdm-2, MEKK-1, NEDD, NF- B, NuMa, p53, PAK-2, PARP, perforin, phosphatidylserine, PITSLRE, PKC , pRb, preseniline, price, RAIDD, Ras, RIP, sphingomyelinase, SREBPs, TNF-, TNF- receptor, TRADD, TRAF2, TRAIL, e.g. TRAIL-R1, TRAIL-R2, TRAIL-R3, or transglutaminase, U1-70 kDa snRNP, YAMA.

6. The conjugate molecule of any of claims 1 to 4, wherein portion (II) is a nucleic acid sequence selected from genomic DNA sequences, subgenomic DNA sequences, cDNA sequences, synthetic DNA sequences, anti-sense oligonucleotides, DNA-enzymes, RNA sequences, siRNA, ribozymes, e.g. hammerhead ribozymes, artificial chromosomes, mini chromosomes and combinations thereof.

7. The conjugate molecule of any of claims 1 to 4, wherein portion (II) is an antibody or an antibody fragment selected from monoclonal antibodies, polyclonal antibodies, polyclonal monospecific antibodies, chimeric antibodies, idiotypic antibodies, anti-idiotypic antibodies, (anti-)anti-idiotypic antibodies, and genetically manipulated antibodies.

8. The conjugate molecule of any of claims 1 to 4, wherein portion (II) is a polymer selected from dextran, pluronics, e.g. polyethyleneoxide (PEO), polypropyleneoxide (PPO), polyethyleneglycol (PEG), poylethyleneimine (PEI), poly[N-(2-hydroxypropyl)methacrylamide] (PHPMA), starch, poly-L-lysine (pLL), poly-R-lysine (pLR), Chitosan, poly-glutamic acid.

9. The conjugate molecule of any of claims 1 to 4, wherein portion (II) is a polymer being part of a pharmaceutical compositions or a liposomal composition.

10. The conjugate molecule of any of claims 1 to 9, wherein portion (II) is labelled for detection with a label selected from radioactive labels, coloured dyes, fluorescent groups, chemoluminiscent groups, immobilizing moieties, or a combination of these labels.

11. The conjugate molecule of any of claims 1 to 10, wherein the conjugate molecule comprises 1 to 10, preferably 1 to 25 and more preferably 1 to 50 portions (I) per portion (II).

12. The conjugate molecule of any of claims 1 to 11, wherein the conjugate molecule additionally comprises an additional portion (III).

13. The conjugate molecule of claim 12, wherein the additional portion (III) allows the inventive conjugate molecule to specifically bind to a preselected cell type.

14. A conjugate molecule of any of claims 1 to 13 as medicament.

15. Use of a conjugate molecule any of claims 1 to 13 for the preparation of a medicament for the treatment, diagnosis and/or prophylaxis of autoimmune diseases, infectious diseases, cancer diseases, neoplastic conditions or tumors or for induction of apoptosis in cells.

16. A method for treating a condition comprising the steps of:
administering a therapeutically effective amount of a conjugate molecule of any of claims 1 to 13.

17. Method of claim 16, wherein the condition is selected from autoimmune diseases, infectious diseases, cancer diseases, neoplastic conditions or tumors.
